# EUROPEAN PATENT APPLICATION

(11) **EP 3 904 378 A1**
(43) Date of publication of application: **03.11.2021**
(21) Application number: 20172168.5
(22) Date of filing: 29.04.2020
(51) Int. Cl.: C07K 16/00, C07K 16/18

(54) **BISPECIFIC PROTEIN**

(71) Applicant: Hober Biotech AB, 115 46 Stockholm (SE)
(72) Inventor: HOBER, Sophia, 115 46 Stockholm (SE); LINDBO, Sarah, 18143 Lidingö (SE); VON WITTING, Emma, 121 49 Johanneshov (SE)
(74) Representative: Kransell & Wennborg KB

(57) **Abstract**

The present disclosure provides for a bispecific protein that enables simultaneous binding of both albumin and a desired target to an ADAPT (ABD Derived Affinity ProTeins) molecule. The present disclosure provides for an ADAPT molecule wherein the proposed binding surface of the desired target is located on helix 1 and helix 2 of the ADAPT molecule.

SEQUENCE LISTING

<110>  HOBER BIOTECH AB

<120>  Bispecific protein

<130>  200267EP

<160>  5

<170>  PatentIn version 3.5

<210>  1
<211>  46
<212>  PRT
<213>  Homo Sapiens



<220>
<221>  misc_feature
<222>  (11)..(11)
<223>  Xaa can be any naturally occurring amino acid, preferably not Cys,
Gly or Pro

<220>
<221>  misc_feature
<222>  (15)..(15)
<223>  Xaa can be any naturally occurring amino acid, preferably not Cys,
Gly or Pro

<220>
<221>  misc_feature
<222>  (17)..(17)
<223>  Xaa can be any naturally occurring amino acid

<220>
<221>  misc_feature
<222>  (19)..(19)
<223>  Xaa can be any naturally occurring amino acid, preferably not Cys,
Gly or Pro

<220>
<221>  misc_feature
<222>  (20)..(20)
<223>  Xaa is selected from Tyr or Phe

<220>
<221>  misc_feature
<222>  (23)..(23)
<223>  Xaa is selected from Asn,Asp or Arg

<220>
<221>  misc_feature
<222>  (26)..(26)
<223>  Xaa is selected from Asn or Asp

<220>
<221>  misc_feature
<222>  (27)..(27)
<223>  Xaa can be any naturally occurring amino acid, may preferably be
selected from Asn, Lys or Asp

<220>

<221>  misc_feature
<222>  (33)..(33)
<223>  Xaa can be any naturally occurring amino acid, may preferably be
selected from Gly, Glu, Ser or Asp

<220>
<221>  misc_feature
<222>  (35)..(35)
<223>  Xaa can be any naturally occurring amino acid, preferably not Cys,
Gly or Pro

<220>
<221>  misc_feature
<222>  (36)..(36)
<223>  Xaa can be any naturally occurring amino acid, may preferably be
selected from Asp, Ala, Ser, Glu or Lys

<220>
<221>  misc_feature
<222>  (38)..(38)
<223>  Xaa can be any naturally occurring amino acid, preferably not Cys,
Gly or Pro

<220>
<221>  misc_feature
<222>  (39)..(39)
<223>  Xaa can be any naturally occurring amino acid, preferably not Cys,
Gly or Pro

<220>
<221>  misc_feature
<222>  (40)..(40)
<223>  Xaa is selected from Glu or His

<220>
<221>  misc_feature
<222>  (43)..(43)
<223>  Xaa can be any naturally occurring amino acid, preferably not Cys,
Gly or Pro, or may be absent

<220>
<221>  misc_feature
<222>  (44)..(44)
<223>  Xaa can be any naturally occurring amino acid, preferably not Cys,
Gly or Pro, or may be absent

<220>
<221>  misc_feature
<222>  (45)..(46)
<223>  Xaa can be any naturally occurring amino acid or absent

<400>  1

Leu Ala Glu Ala Lys Val Leu Ala Asn Arg Xaa Leu Asp Lys Xaa Gly
1               5                   10                  15

Xaa Ser Xaa Xaa Tyr Lys Xaa Leu Ile Xaa Xaa Ala Lys Thr Val Glu
            20                  25                  30

Xaa Val Xaa Xaa Leu Xaa Xaa Xaa Ile Leu Xaa Xaa Xaa Xaa
        35                  40                  45

<210>  2
<211>  81
<212>  DNA
<213>  Artifical sequence

<220>
<221>  misc_feature
<222>  (28)..(33)
<223>  n is a, c, g, or t

<220>
<221>  misc_feature
<222>  (40)..(45)
<223>  n is a, c, g, or t

<220>
<221>  misc_feature
<222>  (49)..(54)
<223>  n is a, c, g, or t

<220>
<221>  misc_feature
<222>  (61)..(66)
<223>  n is a, c, g, or t

<400>  2
gatgaagccg tcgacgccaa ttcactgnnn nnngcgaaan nnnnngcgnn nnnngaactg      60

nnnnnntatg gcgtgagcga t                                                81


<210>  3
<211>  54
<212>  DNA
<213>  Artifical sequence


<220>
<221>  misc_feature
<222>  (20)..(21)
<223>  n is a, c, g, or t

<220>
<221>  misc_feature
<222>  (29)..(30)
<223>  n is a, c, g, or t

<220>
<221>  misc_feature
<222>  (32)..(33)
<223>  n is a, c, g, or t

<400>  3
ttccacggtt ttcgctttmn naatcagmnn mnnataaaaa tcgctcacgc cata            54


<210>  4
<211>  57
<212>  DNA
<213>  Artificial sequence

<220>
<223> Artificially constructed oligonucleotide

<400> 4
cggcagcgcc gccagaatat gcagtttcag cgcttccacg ccttccacgg ttttcgc 57

<210> 5
<211> 54
<212> DNA
<213> Artifical sequence

<220>
<221> misc_feature
<222> (19)..(21)
<223> n is a, c, g, or t

<220>
<221> misc_feature
<222> (28)..(33)
<223> n is a, c, g, or t

<400> 5
ttccacggtt ttcgctttnn naatcagnnn nnnataaaaa tcgctcacgc cata 54

## Description

### TECHNICAL FIELD

The present disclosure relates to the field of engineered bispecific proteins.

### BACKGROUND

Owing to their extraordinary affinity and target specificity, antibodies have been tremendously successful within the pharmaceutical industry. In fact, six out of the ten best-selling drugs on the market today are antibodies [1] and their combined sales were approaching $100 billion at the end of 2017 [2]. Despite their success, antibodies are not without limitations. Their large and complex structure leads to high production costs and suboptimal pharmacokinetics in many clinical applications. In light of these limitations, and thanks to the development and refinement of protein engineering techniques during the latest decades, other alternatives have emerged. Among these are the small affinity protein domains, collectively known as Alternative Scaffolds. These scaffolds have several potential benefits as therapeutics and can be engineered to have high affinity and target selectivity. In addition, their small size, which entails both a low cost of goods and an ability to penetrate tissue that is otherwise inaccessible, makes them an attractive alternative to antibodies.

Apart from affecting the production process and the biodistribution, another potential benefit of the small size is the possibility of using alternative administration routes. For large molecules like antibodies, options are limited to intravenous and subcutaneous injection, both being highly inconvenient for the patient and causing a high burden on the healthcare system [3]. For the much smaller alternative scaffolds, nasal or pulmonary delivery could very well serve as a non-invasive alternative, possibly improving patient convenience and compliance. Previous studies have shown that the molecular mass and size of a protein is the most critical factor influencing the bioavailability of drugs administered via the mucosa and consequently, keeping the size to a minimum becomes especially important [3,4].

The small size also causes rapid clearance from the circulation, a feature that can be both an advantage as well as a limitation depending on the application. Fast blood clearance together with high tissue penetration has been essential to achieve high contrast in molecular imaging applications. In fact, many of the alternative scaffolds (e.g. Affibody, DARPin and ADAPT) have been shown to be very promising as imaging probes, exhibiting extraordinary contrast from surrounding healthy tissue [5-7]. On the other hand, in most therapeutic applications, rapid clearance is unfavorable since a long residence time is required to keep the dosing low and less frequent. A rapid clearance typically also yields undesired accumulation in the kidneys. To overcome this size-related limitation, several strategies for half-life extension have been developed [8]. One of these strategies is based on fusion to the Albumin Binding Domain (ABD) of Streptococcal Protein G. By utilizing the noncovalent association with the patient's own serum albumin, a substantial increase in half-life and decrease in kidney accumulation have been shown for scaffold fusion proteins developed through this strategy [9-11]. The increase of the circulatory half-life is not only attributed to the increase in size of the complex and hence ability to avoid filtration through the glomerular barrier but also to the fact that albumin is rescued from lysosomal degradation by association with the neonatal Fc receptor, FcRn [12]. Although frequently used, one obstacle of most half-life extension strategies that exist today is that they counteract some of the benefits associated with the small size.

With the aim of developing a new strategy for half-life extension, without altering the size of the molecule, a group of researchers including the present inventors has in the past engineered the ABD itself. In this previous work, a novel binding surface was introduced into the ABD, in addition to its native binding to albumin. By randomizing residues in the first and third helix of the ABD, bispecific ADAPTs (ABD Derived Affinity ProTeins) towards the Z2 domain[13], TNFα[14], HER2[15] and HER3[16] were generated.

### SUMMARY

The present inventors observed that binding both albumin and the desired target simultaneously to the ADAPT molecule was not feasible in the previous work, probably due to steric hindrance. Since albumin concentrations in serum is high (∼600µM) [17], even a low affinity towards it would most certainly prevent target binding *in vivo* for these molecules.

Therefore, it is an objective of the present disclosure to provide a bispecific protein that enables simultaneous binding of both albumin and a desired target on the ADAPT molecule. The objective is attained by designing an ADAPT molecule wherein the proposed binding surface of the desired target has been moved from helix 1 and helix 3 to helix 1 and helix 2 of the ADAPT molecule.

To meet at least the above-mentioned objective, according to a first aspect there is provided a bispecific protein capable of binding Human Serum Albumin (HSA) and another target, which bispecific protein comprises the amino acid sequence LAEAKVLANR X11LDKX15GX17SX19X20 YKX23LI X26X27AKT VEX33VX35X36LX38X39X40 ILX43X44X45X46 (SEQ ID No. 1), wherein:
X11, X15, X17, X19, X27, X33, X35, X36, X38 and X39 are, independently of each other, any amino acid;
X43, X44, X45 and X46 are, independently of each other, absent or any amino acid;
X20 is Y or F; X23 is N, D or R;
X26 is N or D; and X40 is E or H;
with the proviso that at least one of positions 22, 23 and 26 and at least one of positions 2, 3, 6, 7, 9, 10, 13 and 14 have been mutated to obtain the affinity for the other target.

According to one embodiment at least two of positions 22, 23 and 26 have been mutated to obtain the affinity for the other target.

According to one embodiment all of positions 22, 23 and 26 have been mutated to obtain the affinity for the other target.

According to one embodiment at least one of positions 2, 9 and 13 have been mutated to obtain the affinity for the other target.

According to one embodiment position 9 has been mutated to obtain the affinity for the other target.

According to one embodiment position X27 is N, K or D; and/or X33 is G, E, S or D.

According to one embodiment X36 is D, A, S, E or K.

According to one embodiment neither of the amino acids in positions 1-15, 19-26 and/or 31-44 are C.

According to one embodiment neither of the amino acids in positions 1-15 and/or 19-26 are G.

According to one embodiment neither of the amino acids in positions 1-15, 19-26 and/or 31-44 are P

According to one embodiment positions 1, 4, 5, 8, 12, 16, 25, 31, 34 and 42, each and independently, are conserved, or are conservatively substituted.

According to a second aspect there is provided a polynucleotide encoding the bispecific protein according any embodiment of the first aspect.

According to a third aspect there is provided an expression cassette comprising the polynucleotide according to the second aspect.

According to a fourth aspect there is provided a vector comprising the polynucleotide according to the second aspect, or the expression cassette according to the third aspect.

According to a fifth aspect there is provided a pharmaceutical composition comprising the bispecific protein according to any embodiment of the first aspect.

According to one embodiment of the fifth aspect the pharmaceutical composition further comprises a target molecule associated, bound or coupled to the bispecific protein.

According to one embodiment the target molecule is chosen from a group comprising of tumor necrosis factor alpha (TNFα), Prostate Specific Antigen (PSA), C-reactive protein (CRP), renin (REN), angiogenin (ANG), myeloid-derived growth factor (MYDGF) and insulin.

According to one embodiment of the fifth aspect the pharmaceutical composition further comprises a pharmaceutically acceptable carrier, excipient, stabilizer, additive, buffer solution, and/or solvent.

According to a sixth aspect there is provided a therapeutic agent comprising the bispecific protein of any of the embodiments of the first aspect, and a cytotoxic agent, such as a cytotoxic molecule, peptide, protein or radionuclide.

According to one embodiment of the sixth aspect the cytotoxic agent is an alkylating drug, anthracycline, an antimetabolite, a vinca alkaloide, an etoposide, an antineoplastic drug or agent, an exotoxin, a ribosome-inactivating protein, or a protein kinase inhibitor.

### BRIEF DESCRIPTION OF THE DRAWINGS

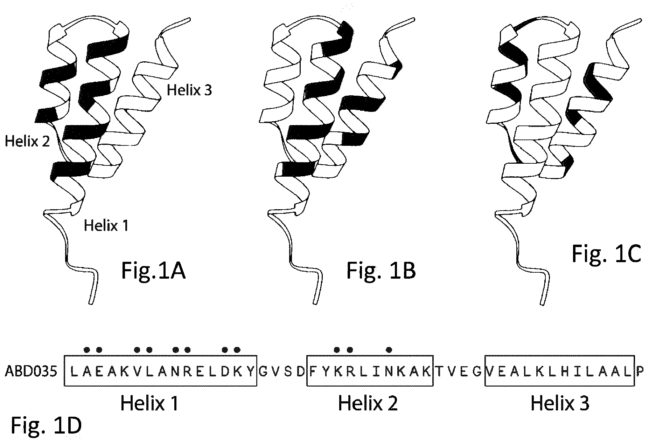

Figure 1A illustrates the design of the new bispecific protein with the randomized positions shown in black. Fig. 1B illustrates the binding surface, shown in black, of previously published bispecific binders that were sterically hindered from binding HSA and other targets simultaneously. Fig. 1C describes the binding towards HSA shown in black. Fig. 1D discloses the sequence of the parental molecule ABD035 and the randomized positions of the present disclosure marked with black dots.

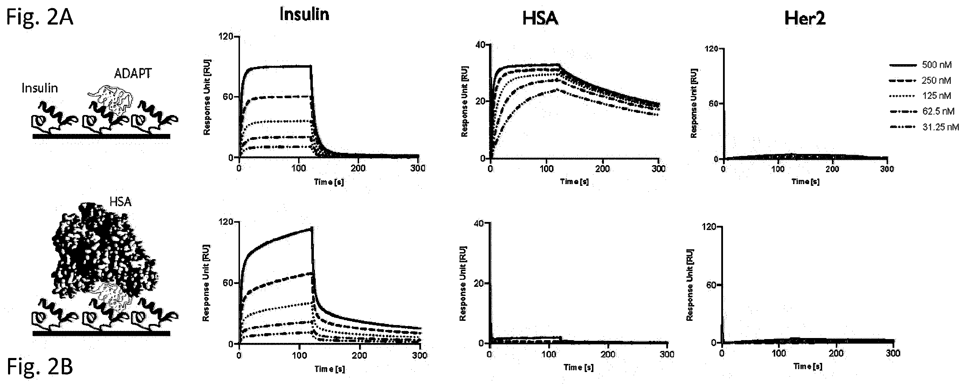

Figure 2 discloses the ability of the bispecific proteins of the present disclosure to bind both HSA and a target simultaneously. Fig. 2A discloses SPR sensorgrams of ADAPT_{Insulin_02} that show binding to Insulin and HSA but not to the irrelevant target Her2. Fig. 2B) discloses the same analysis but with the ADAPT_{Insulin_02} being saturated with HSA, showing that it can still bind Insulin but not HSA.

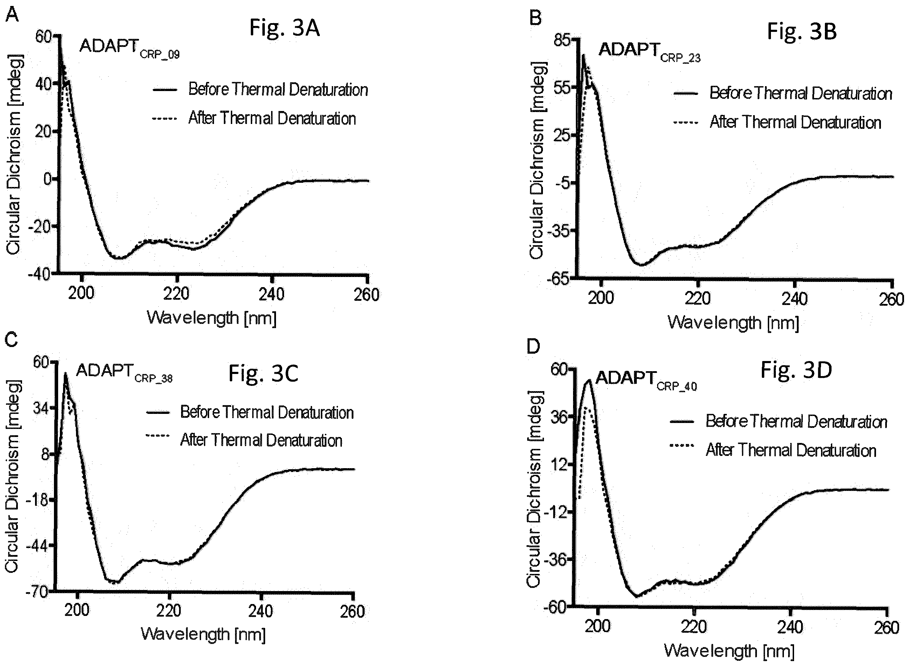

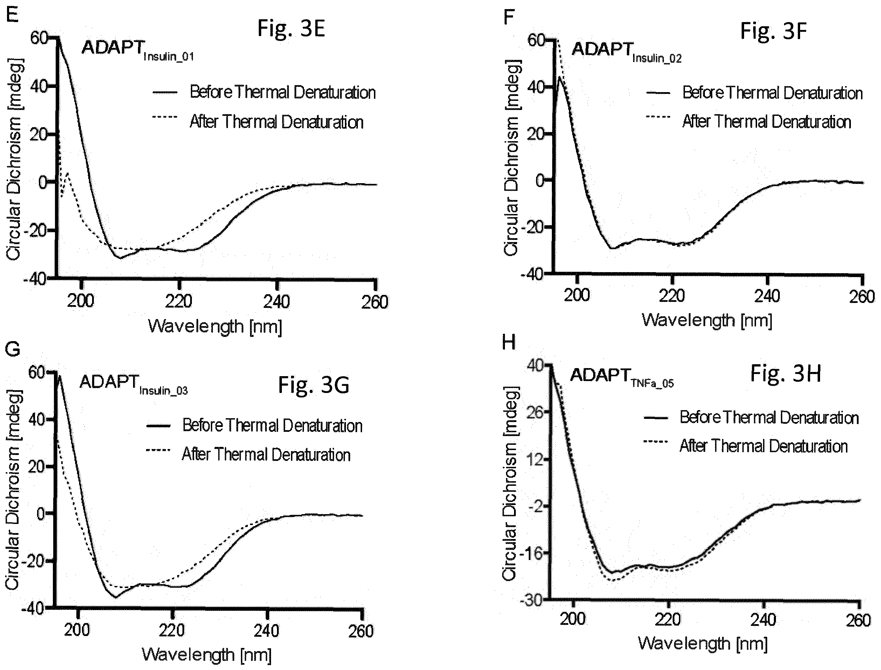

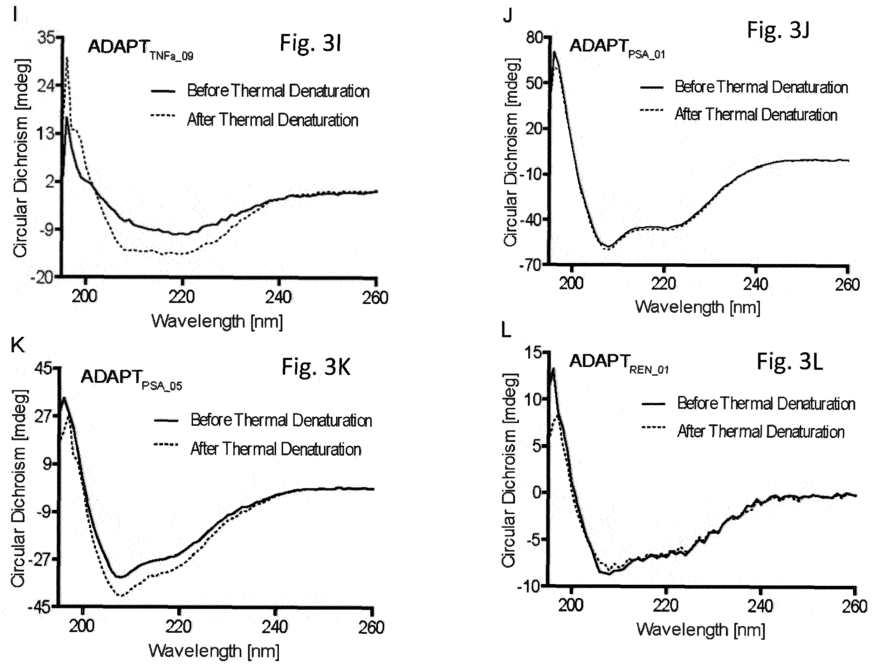

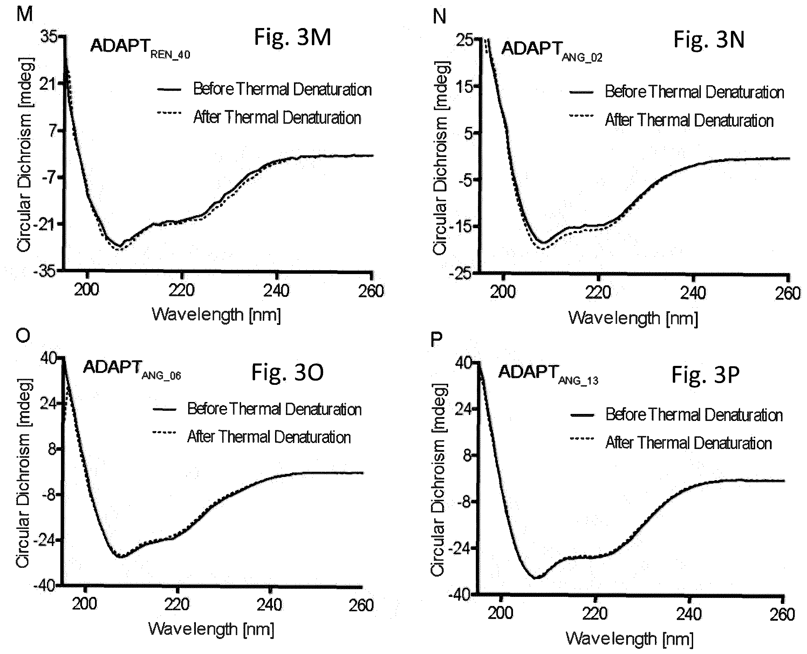

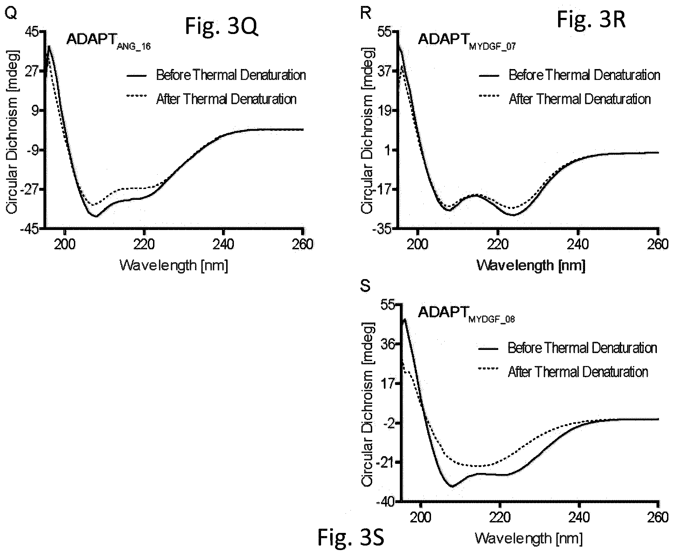

Figure 3 discloses CD spectra of the selected ADAPT variants, before (black line) and after (black dots) thermal denaturation. A) ADAPT_CRP_09; B) ADAPT_CRP_23; C) ADAPT_CRP_38; D) ADAPT_CRP_40; E) ADAPT_Insulin_01; F) ADAPT_Insulin_02; G) ADAPT_Insulin_03; H) ADAPT_TNF_05; I) ADAPT_TNF_09; J) ADAPT_PSA_01; K) ADAPT_PSA_05; L) ADAPT_REN_01; M) ADAPT_REN_40; N) ADAPT_ANG_02; O) ADAPT_ANG_06; P) ADAPT_ANG_13; Q) ADAPT_ANG_16; R) ADAPT_MYDGF_07; S) ADAPT_MYDGF_08

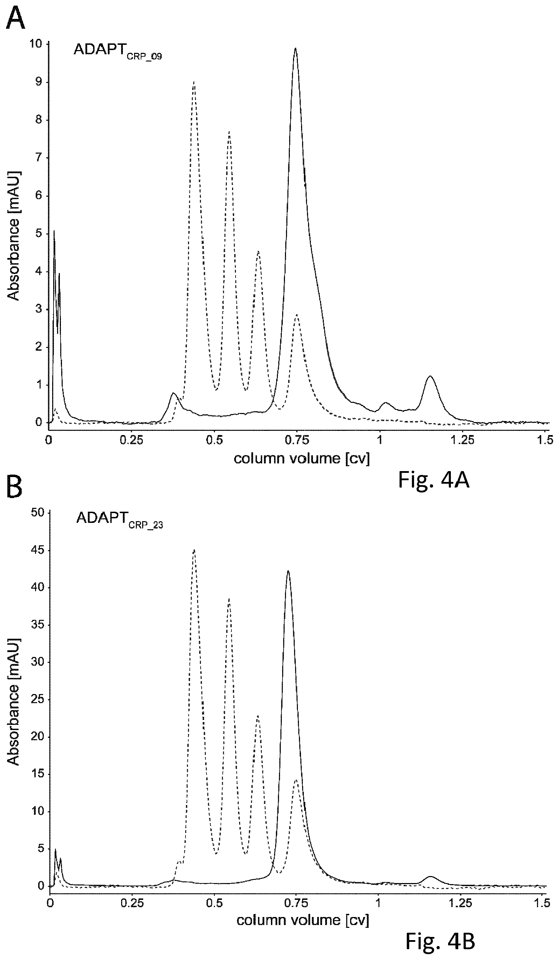

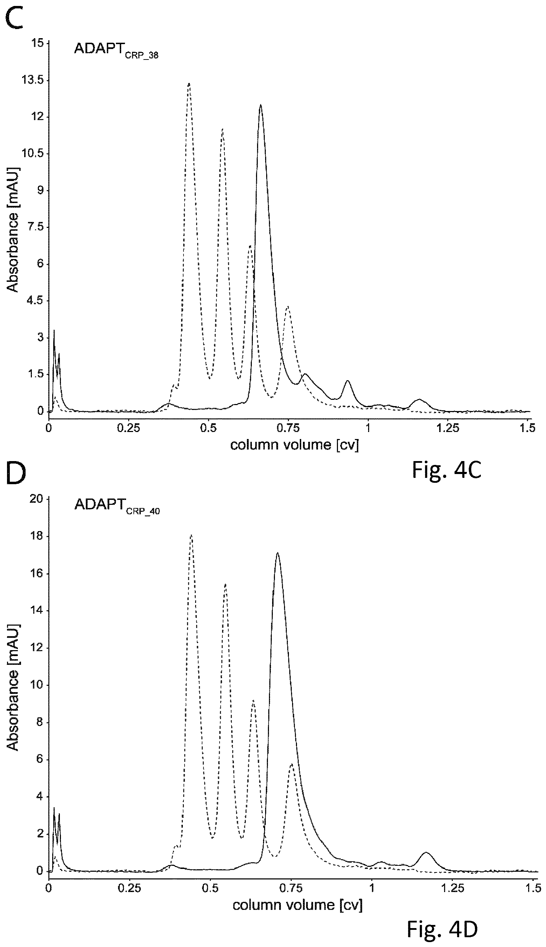

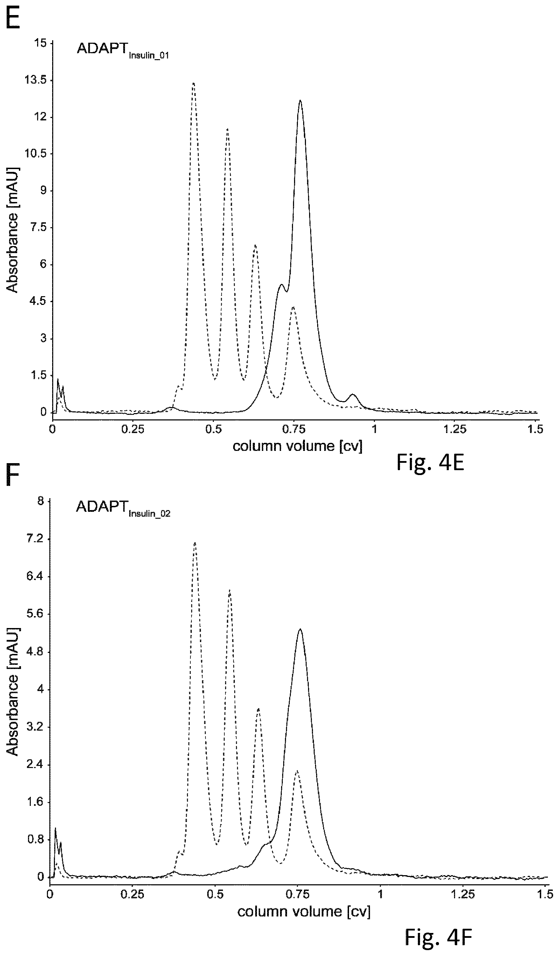

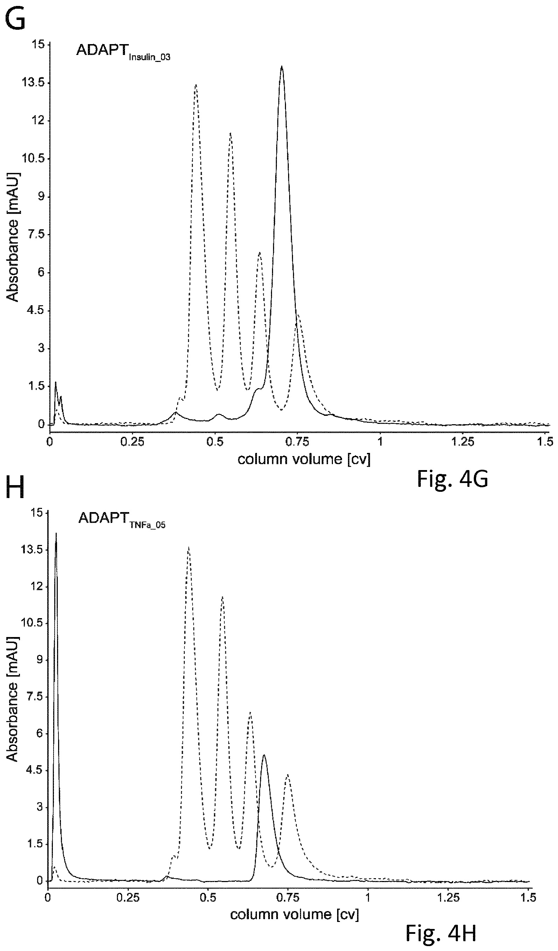

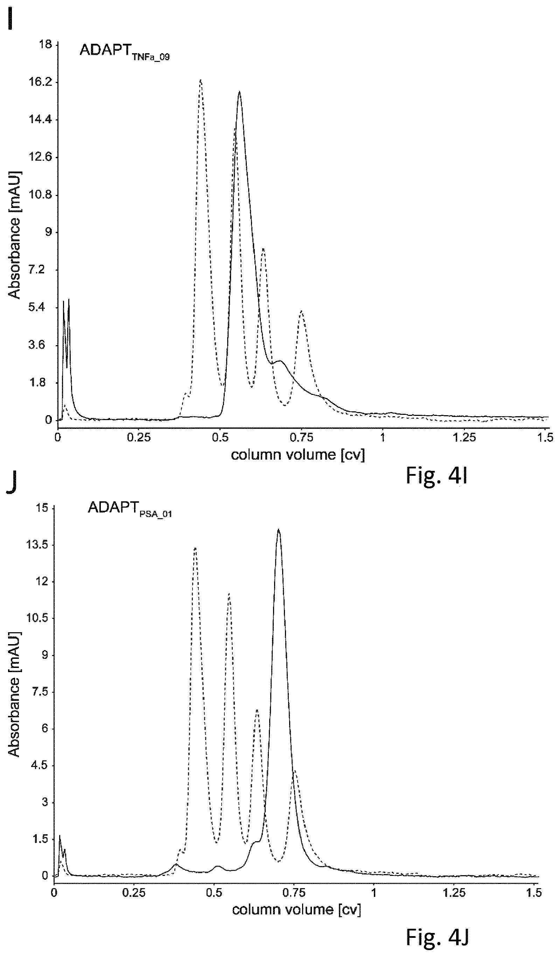

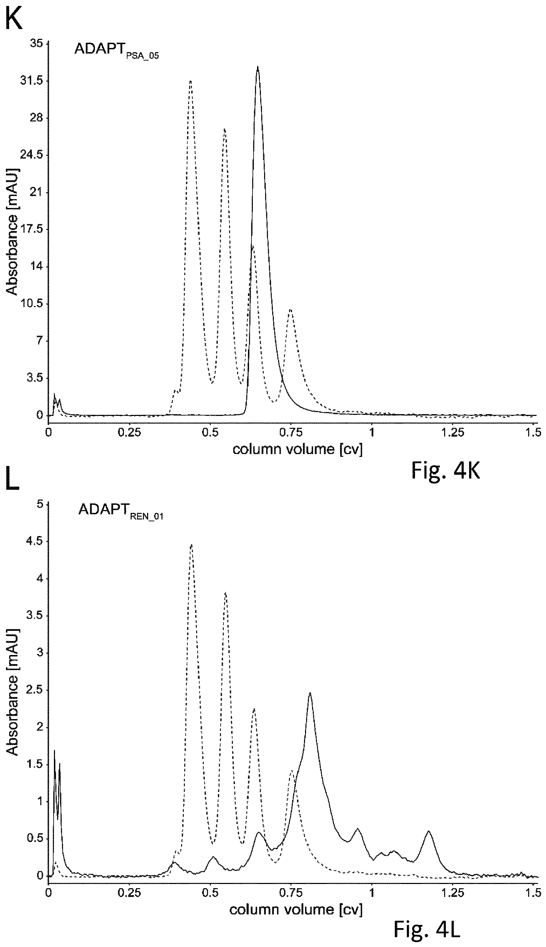

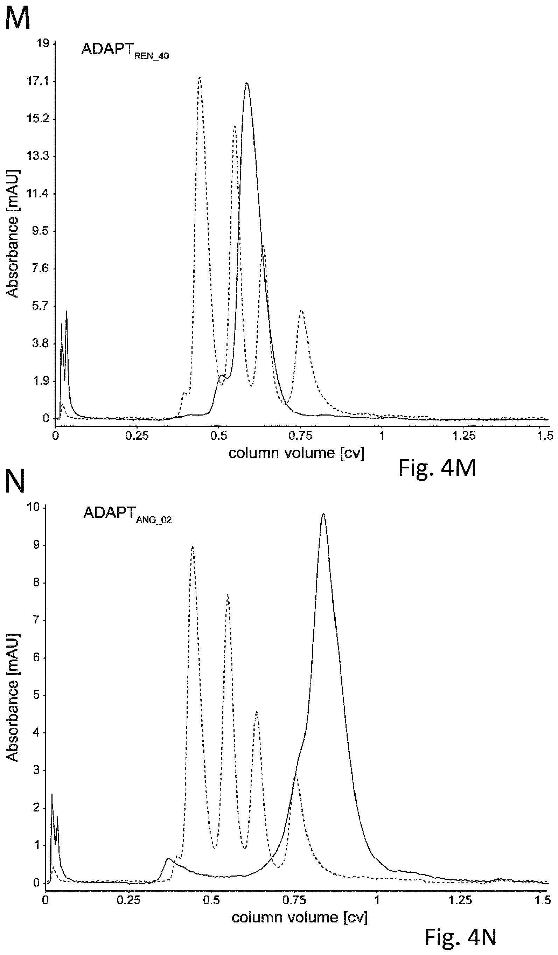

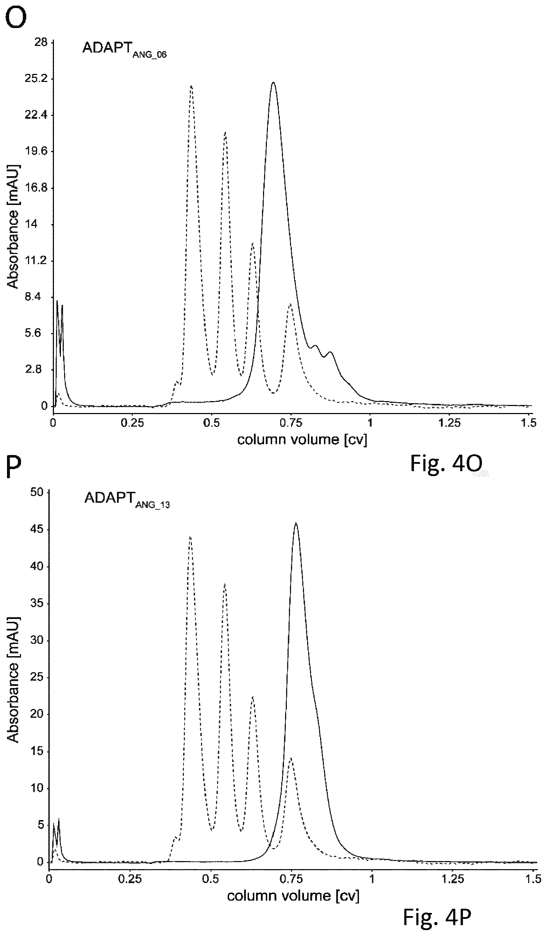

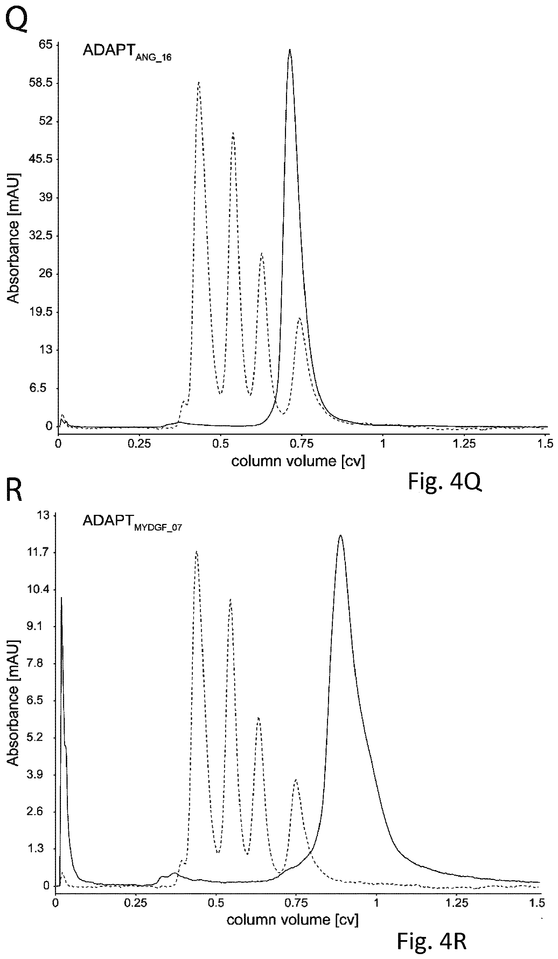

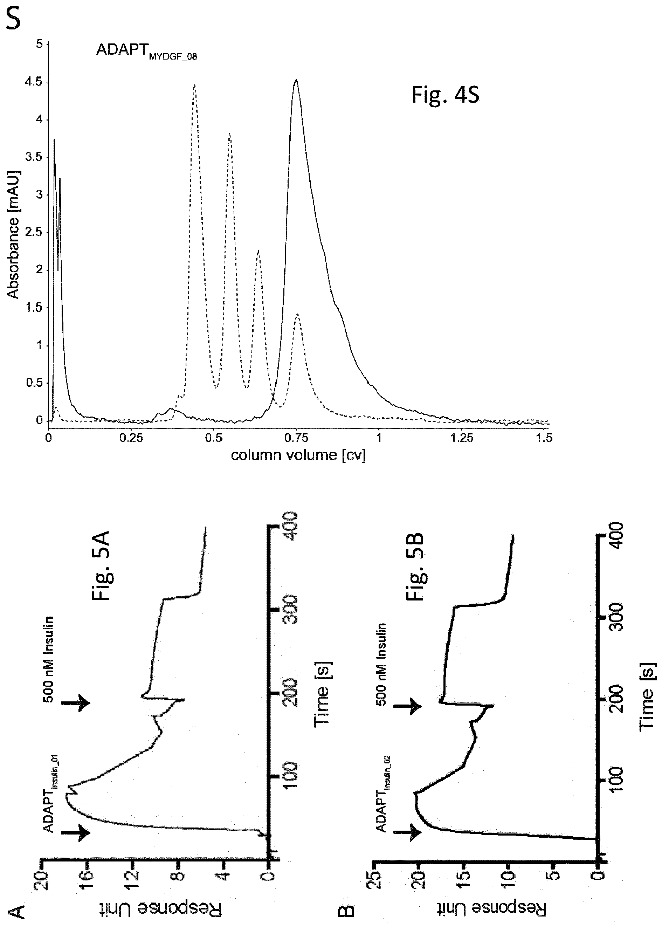

Figure 4 discloses an evaluation of oligomeric state through SEC chromatogram showing selected ADAPT variants (black line) overlaid with calibrant (black dots). A) ADAPT_CRP_09; B) ADAPT_CRP_23; C) ADAPT_CRP_38; D) ADAPT_CRP_40; E) ADAPT_Insulin_01; F) ADAPT_Insulin_02; G) ADAPT_Insulin_03; H) ADAPT_TNF_05; I) ADAPT_TNF_09; J) ADAPT_PSA_01; K) ADAPT_PSA_05; L) ADAPT_REN_01; M) ADAPT_REN_40; N) ADAPT_ANG_02; O) ADAPT_ANG_06; P) ADAPT_ANG_13; Q) ADAPT_ANG_16; R) ADAPT-MYDGF-07; S) ADAPT_MYDGF_08.

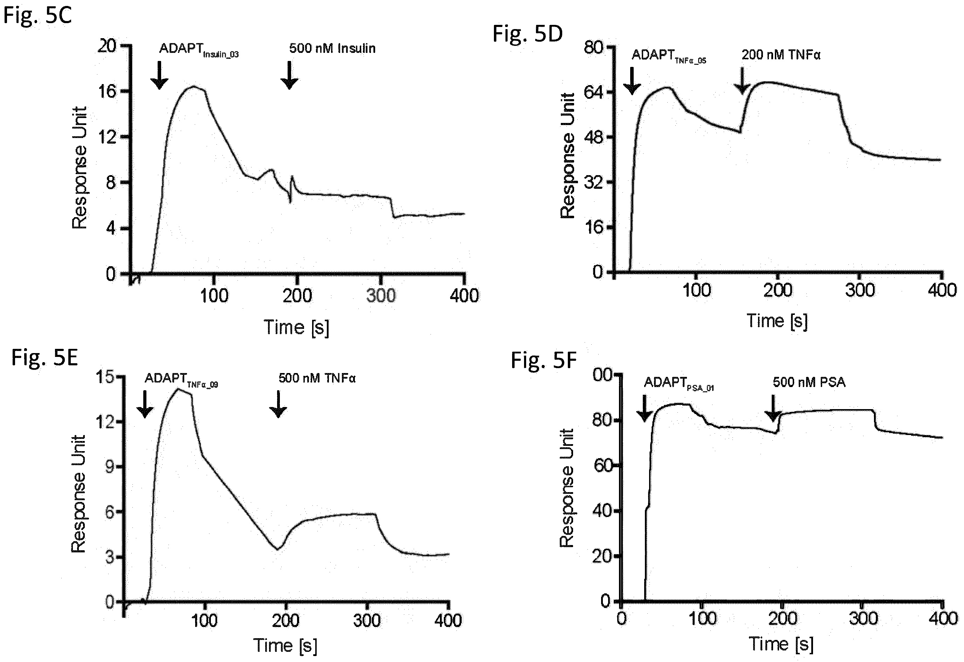

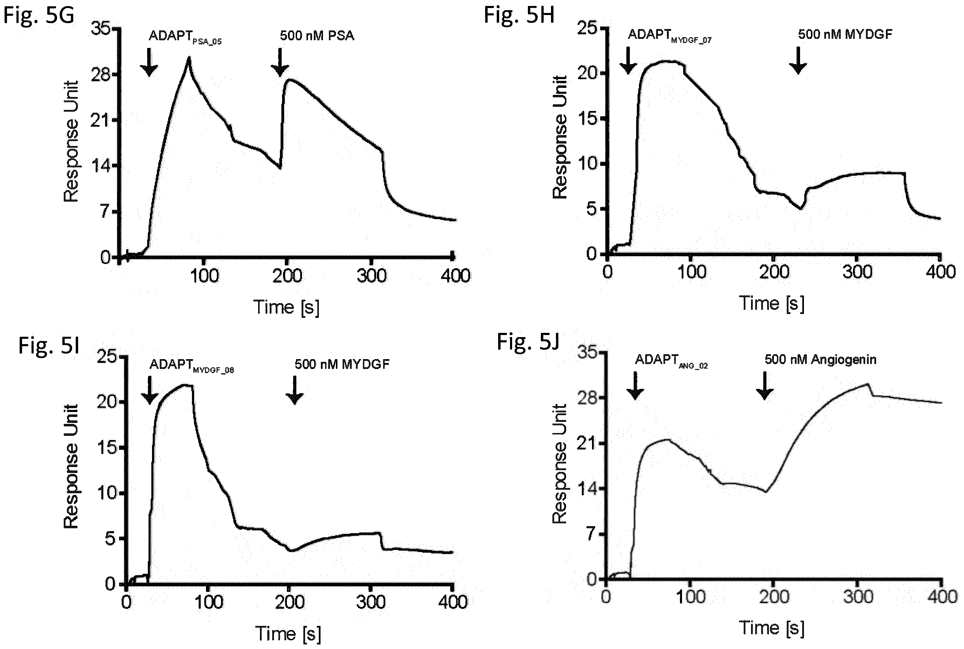

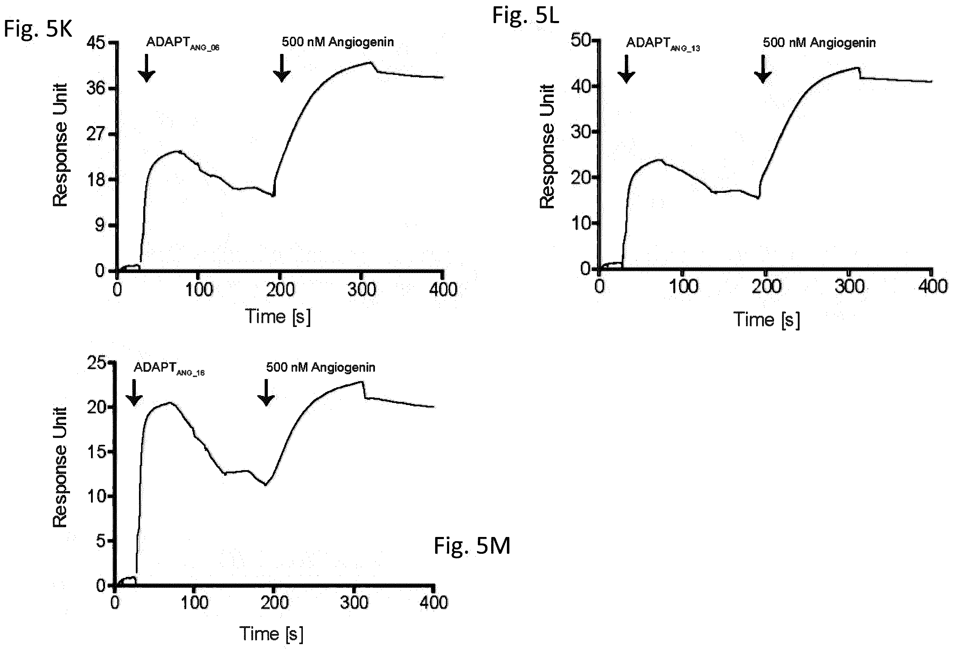

Figure 5 discloses an evaluation of simultaneous binding through SPR Capture assay. SPR sensorgrams of ADAPT variants indicating simultaneous binding of HSA and target by demonstrating binding curvature for injected target after capture on a surface immobilized with HSA. A) ADAPT_Insulin_01; B) ADAPT_Insulin_02; C) ADAPT_Insulin03; D) ADAPT_TNFa_05; E) ADAPT_TNFa_09; F) ADAPT_PSA_01; G) ADAPT_PSA_05; H) ADAPT-MYDGF-07; I) ADAPT_MYDGF_08; J) ADAPT_ANG_02; K) ADAPT_ANG_06; L) ADAPT_ANG_13; M) ADAPT_ANG_16.

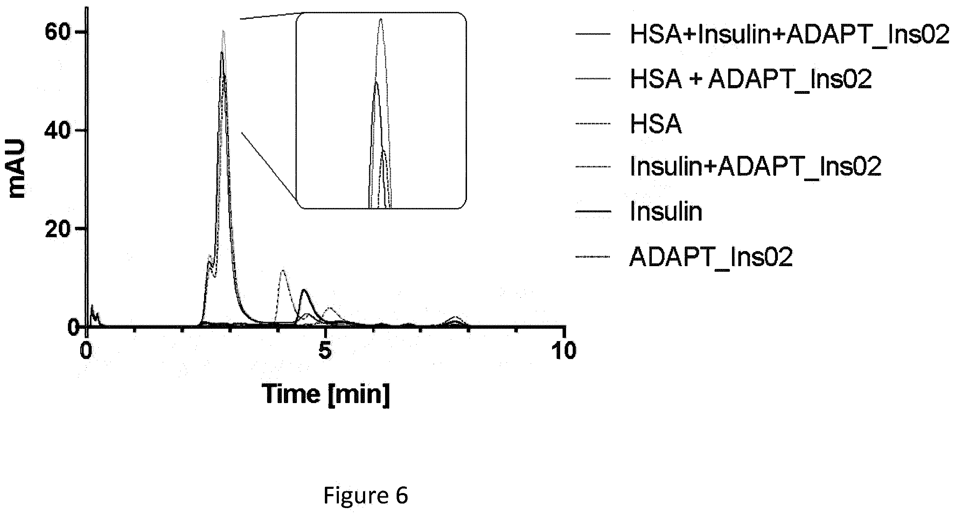

Figure 6 discloses formation of ternary complex as exemplified by ADAPT_Insulin_02. SEC Chromatogram with overlaid signals of individual injections of HSA, ADAPT_Ins02 and Insulin as well as a sample with Insulin and ADAPT_Ins02, a sample with HSA and ADAPT_Ins02 and a sample with all three binding partners.

### DEFINITIONS

"ABD" stands for Albumin binding domain.

"HSA" stands for Human Serum Albumin
The bispecific protein in the present disclosure is also disclosed herein using the term "ADAPT", an abbreviation for ABD Derived Affinity ProTeins, or the term "binder".

"Target" as used herein denotes a target molecule that will bind to the bispecific protein, and which molecule will be the effector molecule in the complex of the bispecific protein bound to said target and to HSA.

For amino acids, the following abbreviations are used: Alanine - A; Arginine - R; Asparagine - N; Aspartic acid - D; Cysteine - C; Glutamic acid - E; Glutamine - Q; Glycine - G; Histidine - H; Isoleucine - I; Leucine - L; Lysine - K; Methionine - M; Phenylalanine - F; Proline - P; Serine - S; Threonine - T; Tryptophan - W; Tyrosine - Y; Valine - V.

### DETAILED DESCRIPTION

Small size is an attractive feature of therapeutic proteins since it allows for high tissue penetration and non-invasive administration routes as well as easy and cost-efficient production. However, advantages often come with a price. These small proteins typically have a limited *in vivo* half-life leading to substantial kidney uptake as well as lower therapeutic efficacy. A well-known strategy to overcome these limitations is to utilize the long half-life of human serum albumin either by direct fusion to albumin itself or to different albumin binding moieties. Previous studies have shown that fusing therapeutic molecules to an ABD derived from Streptococcal Protein G leads to substantial decrease in kidney uptake and considerably longer half-lives [10,23].

In the study underlying the present disclosure, the albumin binding properties of ABD have been utilized, but instead of fusing it to another binder, an additional binding site has instead been introduced in the molecule itself. Hence, small single-domain bispecific binders with improved pharmacokinetic properties are achieved. To do this, a novel combinatorial phage display library was designed and used to select molecules with the aim of binding both HSA and other clinically relevant targets at the same time.

It has previously been confirmed that it is possible to introduce dual binding specificity into this scaffold [13-16]. However, in these studies, binding of one of the partners somehow prevented simultaneous binding of the other one. The present inventors hypothesized that this might be due to steric hindrance and therefore designed the library disclosed herein with the novel binding surface on the opposite side of the molecule (See Figure 1). This proved to be a successful strategy as it resulted in several bispecific molecules exhibiting simultaneous binding.

The present inventors have thus designed a new combinatorial library where the proposed binding surface has been moved to Helix 1 and 2 of the ABD. The hypothesis was that the novel library design would increase the possibility of generating minimal proteins capable of binding albumin and other targets simultaneously, thereby combining the advantages of both small and large therapeutic molecules in one single format. To demonstrate the quality and applicability of this novel combinatorial library, phage display selections were performed towards a panel of clinically relevant targets of different size (TNFα, PSA, CRP, REN, ANG, MYDGF and Insulin). In addition, a standardized workflow including next generation sequencing and a PCR-based method for gene recovery was designed to allow for cost-efficient isolation of target-binding clones. The results from the present study showed that the novel binding surface was suitable for target interactions and also enabled simultaneous binding to serum albumin.

Hence is provided a bispecific protein capable of binding Human Serum Albumin (HSA) and another target simultaneously. The bispecific protein comprises the amino acid sequence LAEAKVLANR X11LDKX15GX17SX19X20 YKX23LI X26X27AKT VEX33VX35X36LX38X39X40 ILX43X44X45X46 (SEQ ID No.1). In said sequence, X11, X15, X17, X19, X27, X33, X35, X36, X38 and X39 are, independently of each other, any amino acid. X43, X44, X45 and X46 are, independently of each other, absent or any amino acid. X20 is Y or F. X23 is N, D or R. X26 is N or D. X40 is E or H. At least one of positions 22, 23 and 26 and at least one of positions 2, 3, 6, 7, 9, 10, 13 and 14 in the above-mentioned amino acid sequence have been mutated to obtain the affinity for the other target.

The positions of the amino acids discussed herein for the bispecific protein are referred to in relation to SEQ ID No. 1. For example, when a mutation for the amino acid in position 2 is discussed, it relates to the amino acid in position 2 in SEQ ID No. 1; when a mutation for the amino acid in position 3 is discussed, it relates to the amino acid in position 3 in SEQ ID No. 1; when a mutation for the amino acid in position 22 is discussed, it relates to the amino acid in position 22 in SEQ ID No. 1; and so forth. The protein of SEQ ID No. 1 may be incorporated into a larger complex, with additional amino acids either C-terminally or N-terminally of the SEQ ID No. 1. However, positions of the amino acids discussed herein are always referred to in relation to SEQ ID No. 1 as clarified above.

The bispecific protein according to the above structurally comprises three helices, wherein amino acids 1-15 form helix 1, amino acids 19-26 form helix 2, and amino acids 31-44 form helix 3. Thus, any mutation in at least one of positions 22, 23 and 26 is located in helix 2, and any mutation in at least one of positions 2, 3, 6, 7, 9, 10, 13 and 14 is located in helix 1. The positions 2, 3, 6, 7, 9, 10, 13 and 14 constitute the target binding surface of helix 1, and the positions 22, 23 and 26 constitute the target binding surface of helix 2.

By applying randomized mutations for the specified amino acid positions in helices 1 and 2, and subsequent selection for the above-mentioned targets, the inventors have identified specific amino acid positions that are particularly important for mutation in order to achieve an affinity for the selected target, while still maintaining the stability of the protein and assuring that binding to the target can occur simultaneously with binding to HSA. The specific amino acid positions are located such that the binding site for the target will not sterically hinder the binding of the HSA. See Fig. 1, which clearly illustrates that said specific amino acid positions, being subject to mutations, are directed towards the opposite side of the protein in relation to the HSA binding site.

Furthermore, said specific amino acid positions are located such that they will not interfere with or disturb the stability of the protein.

According to one preferred embodiment, at least two of positions 22, 23 and 26 have been mutated to obtain the affinity for the other target. For instance, positions 22 and 26, or positions 22 and 23, or positions 23 and 26, may have been mutated. According to yet another preferred embodiment, all of positions 22, 23 and 26 have been mutated to obtain the affinity for the other target.

According to one preferred embodiment, at least one of positions 2, 9 and 13 have been mutated to obtain the affinity for the other target. According to yet another preferred embodiment, position 9 has been mutated to obtain the affinity for the other target.

According to one embodiment position X27 is N, K or D. According to one embodiment X33 is G, E, S or D. According to yet another embodiment X36 is D, A, S, E or K.

According to one preferred embodiment neither of the amino acids in positions 1-15, 19-26 and/or 31-44 are C. C is a disulphide forming amino acid, and therefore not appropriate as an undesired disulphide bond will negatively impact the overall structure of such a small protein.

According to one preferred embodiment neither of the amino acids in positions 1-15 and/or 19-26 are G. G is a very flexible amino acid. An increased flexibility may negatively impact the helix structure.

According to one preferred embodiment neither of the amino acids in positions 1-15, 19-26 and/or 31-44 are P. P has a very rigid structure, has helix breaking properties and is therefore disadvantageous for the parts of the protein that form the helix structures.

According to one preferred embodiment, neither of the amino acids in positions 1-15, 19-26 and/or 31-44 are any of C, G or P.

According to one embodiment the amino acids in positions 1, 4, 5, 8, 12, 16, 25, 31, 34 and 42, each and independently, are conserved, or are conservatively substituted. Preferably, the amino acids in positions 1, 4, 5, 8, 12, 16, 25, 31, 34 and 42 are conserved. These positions are important for the stability of the protein and should therefore not be mutated, or alternatively only comprise conservative mutations, so as to maintain and not disturb the structure and stability of the protein.

Conservative substitutions are amino acid replacements that change a given amino acid to a different amino acid with similar physical-biochemical properties. In general, V may be conservatively substituted with A or F; I may be conservatively substituted with F or C; L may be conservatively substituted with F or V; M may be conservatively substituted with F or V; F may be conservatively substituted with L or M; C may be conservatively substituted with V or M; and W may be conservatively substituted with F or L.

In the present disclosure, the inventors have in more detail studied the bispecific protein according to the above, mutated for a higher affinity against targets chosen from a group comprising of tumor necrosis factor alpha (TNFα), Prostate Specific Antigen (PSA), C-reactive protein (CRP), renin (REN), angiogenin (ANG), myeloid-derived growth factor (MYDGF) and insulin. When studying the bispecific protein variants mutated for binding to the above-mentioned targets, the following amino acids were found in the below indicated positions in Table 1.

**Table 1. Alignment of mutated positions in new binders**

| **Amino acid Position** | **2** | **3** | **6** | **7** | **9** | **10** | **13** | **14** | **22** | **23** | **26** |
|---|---|---|---|---|---|---|---|---|---|---|---|
| TNFα_05 | I | W | A | E | V | Q | F | M | Y | D | I |
| TNFα_09 | I | W | A | S | V | R | H | R | Y | E | V |
| CRP_09 | R | V | S | M | W | H | W | S | L | R | D |
| CRP_23 | S | K | A | Q | W | H | Y | Y | L | S | D |
| CRP_38 | K | V | I | I | F | H | W | E | L | S | D |
| CRP_48 | H | Y | I | M | F | H | W | Q | L | Q | D |
| PSA_01 | T | V | F | W | L | T | Q | E | A | N | T |
| PSA_05 | F | R | V | W | V | W | D | Q | D | E | W |
| REN_01 | Y | H | W | M | S | L | A | K | W | N | S |
| REN_40 | M | W | A | E | A | I | V | D | Q | Y | S |
| ANG_02 | Y | W | D | Y | D | Y | W | Q | R | S | W |
| ANG_06 | W | A | D | D | D | W | W | Q | R | A | W |
| ANG_13 | H | E | D | W | D | Y | W | S | R | A | W |
| ANG_16 | Y | V | D | N | D | W | W | Q | R | S | W |
| MYDGF_07 | W | W | V | W | V | A | Q | K | F | K | D |
| MYDGF_08 | W | W | V | S | I | K | Q | A | Y | M | T |
| INS_01 | V | I | A | Y | Q | Y | A | F | S | D | K |
| INS_02 | A | I | A | Y | Q | Y | S | F | R | E | V |
| INS_03 | A | V | A | Y | K | Y | A | F | N | E | L |

Hence, it is clear that the amino acid mutations in the positions specifically identified herein differ depending on the target. It is also clear that the mutations directed towards the same target have large similarities and many features in common, with regards to type of amino acid in a specific position, or even the same amino acid in a specific position. This clearly supports that the amino acid positions specified to be mutated in the bispecific protein of the present disclosure for increased affinity against a target are indeed important. The amino acid positions specified to be mutated in the bispecific protein of the present disclosure are "hot spots" for mutation for the purpose of the present disclosure. The characterization of the above identified mutants is disclosed below in Table 3 under the results section.

Within the present disclosure there is also provided a polynucleotide encoding the bispecific protein according any embodiment of the first aspect. The sequence of the polynucleotide may be codon optimized for expression in a suitable organism or expression system.

The present disclosure also provides an expression cassette comprising the polynucleotide according to the above. The expression cassette comprises the polynucleotide according to the above in an Open Reading Frame (ORF), a promoter and a 3' untranslated region, said region optionally being a polyadenylation site. The promoter is chosen in order to enable expression in a suitable organism or expression system. The expression cassette may comprise additional regulatory sequences.

Furthermore, there is provided a vector comprising the polynucleotide according to the above, or the expression cassette according to the above. The vector may additionally comprise suitable promotors, labels, detectable markers, and/or other regulatory sequences controlling replication and/or expression of the vector in a suitable organism or expression system.

Within the present disclosure there is also provided a pharmaceutical composition comprising the bispecific protein according to any embodiment above.

According to one embodiment the target molecule is chosen from a group comprising of tumor necrosis factor alpha (TNFα), Prostate Specific Antigen (PSA), C-reactive protein (CRP), renin (REN), angiogenin (ANG), myeloid-derived growth factor (MYDGF) and insulin.

According to one embodiment the pharmaceutical composition further comprises a pharmaceutically acceptable carrier, excipient, stabilizer, additive, buffer solution, and/or solvent.

The pharmaceutical composition is preferably adapted for nasal or pulmonary administration. As discussed above in the background section, the size of a molecule or protein is important for the administration thereof, where a smaller size enables using other administration routes, as compared to a larger size. The herein disclosed bispecific protein is small, approx. 6 kDa, and is therefore appropriate for administration via the nasal or pulmonary administration route. By nasal administration, the bispecific protein of the present disclosure may quickly transfer across a single epithelial cell layer in the nasal cavity mucosa and directly enter the systemic blood circulation. This enables a rapid onset for the pharmaceutical agent administered. Furthermore, in general, nasal administration is easy, non-invasive, and comfortable for the subject to be treated or administered the pharmaceutical composition. Thus, the pharmaceutical composition may preferably be formulated in a solution, a suspension or a powder for spraying or dripping into the nose and the nasal cavity. Such pharmaceutical formulations normally may, in addition to the components mentioned above for a pharmaceutical composition, comprise a solubilizer, buffer component, antioxidant, humectant, gelling/viscosifying agent, and taste and/or flavoring masking agent.

The present disclosure also provides a therapeutic agent comprising the bispecific protein of any of the embodiments above, and a cytotoxic agent, such as a cytotoxic molecule, peptide, protein or radionuclide.

According to one embodiment the cytotoxic agent is an alkylating drug, anthracycline or other cytotoxic antibiotic, an antimetabolite, vinca alkaloide, etoposide, an antineoplastic drug or agent, an exotoxin, a ribosome-inactivating protein, or a protein kinase inhibitor.

Examples of an alkylating drug are bendamustine, busulfan, carmustine, chlorambucil, cyclophosphamide, estramustine, ifosfamide, lomustine, melphalan, thiotepa and treosulfan.

Examples of anthracyclines and other cytotoxic antibiotics are bleomycin, calicheamicin, dactinomycin, daunorubicin, doxorubicin, epirubicin, idarubicin, mitomycin, and mitoxantrone.

Examples of antimetabolites are azacitidine, capecitabine, cladribine, clofarabine, cytarabine, fludarabine, fluorouracil, gemcitabine, mercaptopurine, methotrexate, pemetrexed, raltitrexed, and thioguanine.

Examples of vinca alkaloids are vinblastine, vincristine, vindesine, and vinorelbine.

Examples of antineoplastic drugs or agents are α-amanitin, amsacrine, bexarotene, bortezomib, carboplatin, cetuximab, cisplatin, crisantaspase, cryptophycins, dacarbazine, docetaxel, hydroxycarbamide (hydroxyurea), irinotecan, mertansine (emtansine), monomethyl auristatin E, monomethyl auristatin F, oxaliplatin, paclitaxel, pentostatin, procarbazine, ravtansine (soravtansine), rhizoxin, temozolomide, topotecan, tubulysin, trastuzumab, and tretinoin.

Examples of exotoxins are diphtheria toxin, pseudomonas exotoxin A and cholix toxin

Examples of ribosome-inactivating proteins are abrin, beetin, bouganin, gelonin, ricin, saporin, shiga toxin, spiroplasma proteins, sportin and trichosanthin

Examples of protein kinase inhibitors are dasatinib, erlotinib, everolimus, imatinib, nilotinib, and sunitinib.

According to one embodiment, the cytotoxic radionuclide is selected from the group consisting of ¹⁷⁷Lu, ⁹⁰Y, ¹⁸⁸Re_{;} ¹⁸⁶Re;¹⁶⁶Ho, ¹⁵³Sm, ⁶⁷Cu, ⁶⁴Cu, ¹⁴⁹Tb, ¹⁶¹Tb, ⁴⁷Sc; ²²⁵Ac; ²¹²Pb; ²¹³Bi, ²¹²Bi, ²²⁷Th, ²²³Ra; ^{58m}Co, ¹³¹I, ⁷⁶As, ⁷⁷As and ²¹¹At.

In one embodiment, the radionuclide is a radiometal and the bispecific protein is conjugated to the radiometal by a chelator that is covalently bound to the bispecific protein, preferably to a C residue of the bispecific protein. Accordingly, an embodiment of the bispecific protein further comprises a terminal cysteine C residue, preferably a C-terminal C residue. The terminal C residue is preferably the only C residue of the bispecific protein. Said terminal C residue is preferably located C-terminally of the SEQ ID No. 1, and hence not included in the SEQ ID No. 1.

The chelator may be selected from the group consisting of dodecane tetraacetic acid (DOTA) and its derivatives (e.g. the maleimido-derivative of DOTA), cross-bridged macrocyclic chelators and sterically-restricted acyclic chelators.

Particularly suitable chelators for ¹⁷⁷Lu, ⁹⁰Y, ¹⁶⁶Ho, ¹⁵³Sm, ¹⁴⁹Tb, ¹⁶¹Tb, ⁴⁷Sc; ²²⁵Ac; ²¹²Pb; ²¹³Bi, ²¹²Bi, ²²⁷Th and ^{58m}Co are DOTA and its derivative DOTAGA.

For ⁶⁷Cu and ⁶⁴Cu a cross-bridged chelator, such as CB-TE2A, is a better option.

For ¹⁸⁸Re and ¹⁸⁶Re a chelator based on a cysteine- or mercaptoacetyl-containing peptide is preferred.

¹³¹I, ⁷⁶As, ⁷⁷As and ²¹¹At are non-metal radionuclides that can be bound to the bispecific protein by means of covalent conjugation.

The radioiodination may be achieved using ((4-hydroxyphenyl)ethyl) maleimide (HPEM), which can be bound to a C residue of the bispecific protein. ⁷⁶As and ⁷⁷As (and ⁷⁴As) in As (III) form may be coupled directly to a (freshly) reduced thiol group of a C residue of the bispecific protein.

For coupling of ²¹¹At, N-[4-(tri-n-butylstannyl) phenethyl]-maleimide can be used as a linker. In such case, ²¹¹At is first coupled to the linker by astatodestannylation forming 4-astato-phenethyl-maleimide (AtPEM), which in turn can be coupled to a cysteine (C) residue of the fusion protein.

### EXAMPLES - MATERIALS AND METHODS

### Construction of phage display library.

A phagemid vector (pSE) was constructed based on the phagemid pAffi1 [18]. In pSE, the gene fragment encoding helix 3 of protein Z present in pAffi1 was removed and the gene fragment encoding ABDwt was replaced to instead encode the full-length protein Z. Phagemid pSE was ordered from Thermo Fisher Scientific GENEART GmbH (Regensburg, Germany). The restriction enzyme recognition sites for *XhoI* and *NheI,* positioned upstream of Zwt, were used for subsequent insertion of the library gene cassette.

The first ADAPT library gene was assembled from three separate oligonucleotides: fwdABDlib_h1 (SEQ ID No. 2) covering Helix 1 and containing eight diversified positions allowing for all amino acids except Pro, Cys and Gly (Ella Biotech, Martinsried, Germany), revABDlib_h2 (SEQ ID No. 3) covering Helix 2 and containing three positions diversified through NNK-degeneracy (TAG Copenhagen, Copenhagen, Denmark) and revABDlib_h3 (SEQ ID No. 4) covering Helix 3 without any diversified positions (TAG Copenhagen, Copenhagen, Denmark). A second version of the library was assembled by exchanging revABDlib_h2 (SEQ ID No. 3) for revABDlib_h2_v2 (SEQ ID No. 5) (Ella Biotech, Martinsried, Germany), with the same positions but instead diversified to allow all amino acids except Pro, Cys and Gly, just like for Helix 1. Complete oligo sequences can be found in Table 2. 800 pmol of each oligo were mixed at an equal ratio, assembled by 10 cycles of PCR and amplified for another 10 cycles using external primers. These primers also introduced restriction sites XhoI and NheI for ligation into the phagemid vector pSE. The ligated phagemid was extracted from a 2% Agarose gel and purified using QIAquick gel extraction kit (Qiagen, Hilden, Germany). The product was transformed by electroporation into XL1 blue E. coli (Lucigen, Middleton, WI, USA) yielding 5 x 10⁹ members.

**Table 2. Oligos used for library construction of the ADAPT libraries. Sequences of the four oligos used to assemble the libraries. NNN in fwdABDlib_h1 and revABDlib_h2_v2 corresponding to codons allowing all amino acids except Pro, Cys and Gly in equal distribution. MNN in revABDlib_h2 corresponding to NNK degeneracy codons but in reverse order.**

| **Oligo** | **Sequence** |
|---|---|
| fwdABDlib_h1 | GATGAAGCCGTCGACGCCAATTCACTGNNNNNNGCGAAANN NNNNGCGNNNNNNNGAACTGNNNNNNTATGGCGTGAGCGA T |
| revABDlib_h2 | TTCCACGGTTTCGCTTTMNNAATCAGMNNMNNATAAAAAT CGCTCACGCCATA |
| revABDlib_h3 | CGGCAGCGCCGCCAGAATATGCAGTTTCAGCGCTTCCACGCC TTCCACGGTTTTCGC |
| revABDlib_h2_v2 | TTCCACGGTTTCGCTTTNNNAATCAGNNNNNNATAAAAATC GCTCACGCCATA |

### Production and biotinylation of target antigen.

All target antigens except Insulin were produced in the Human Secretome Project (Stockholm, Sweden) as previously described [19]. Human recombinant Insulin was ordered from Thermo Scientific (Waltham, MA, USA). For biotinylation of target antigens, EZ-link Sulfo-NHS-LC-Biotin (Thermo Scientific, Waltham, MA, USA) was dissolved in water to a final concentration of 10 mM and a 12-fold molar excess was added to the target antigen solution. The reaction was incubated at room temperature (RT) for 30 min. Non-reacted biotin reagent was removed from the sample by a NAP5 desalting column (GE Healthcare, Stockholm, Sweden).

### Phage display selections.

Four rounds (two rounds for the Insulin selection) of panning were performed using a biotinylated recombinant target antigen (bTNFα, bCRP, bPSA, bREN, bANG, bMYDGF, bInsulin) in-solution followed by capture on streptavidin-coated magnetic beads. To get rid of non-specifically binding phages, all tubes and beads used during the selection were washed with Phosphate-buffered Saline with 0.1% Tween-20 (PBS-T) and blocked with PBS-T supplemented with 0.5% gelatin. Furthermore, all panning rounds were preceded by an incubation with 0.5 mg of the washed and blocked streptavidin coated magnetic beads in RT at 150 rpm for 30 min. The resulting supernatant was then used as input in the panning rounds. During panning, the phage supernatants were incubated with 150, 100, 50 and 25 nM target antigen for rounds 1-4, respectively. The incubation was allowed to proceed for 2 hours under rotation before capture on streptavidin-coated magnetic beads. The beads were washed twice with PBS-T in round 1, four times in round 2, eight times in round 3 and twelve times in round 4. The bound phages were eluted with 50 mM Glycine pH 2.0 at RT for 10 min followed by neutralization with an equal volume of 10% 1M Tris-HCl in PBS pH 8.0. The neutralized eluates were then used to infect at least a 100x excess of XL1 blue *E. coli* cells compared to the number of eluted phages. After allowing the infection to proceed for 30 minutes, an equal amount of tryptic soy broth with yeast extract (TSB+YE) medium supplemented with 200 µg/ml Ampicillin and 10 µg/ml Tetracycline was added to the cells. 30 minutes later, a 10x excess of M13K07 helper phages, compared to the number of cells used at infection, were added. After an additional 90 min, the cells were pelleted and resuspended in 100 ml TSB+YE supplemented with 100 µg/ml Ampicillin, 25 µg/ml Kanamycin and 0.1 mM isopropyl β-D-1-thiogalactopyranoside (IPTG) and finally incubated overnight at 30°C. The amplified phages were collected by Polyethylene Glycol (PEG) precipitation and used as input in the next panning round.

### Next generation sequencing.

Phagemid DNA was extracted from XL1 blue *E. coli* infected with phage eluates from the different panning rounds or the naive phage library using a QIAprep Miniprep kit (Qiagen, Hilden, Germany). A PCR was performed with 50 ng purified phagemid as template and 5 pmol of an oligo containing the forward adapter sequence and 5 pmol of an oligo containing the reverse adapter sequence as well as a sample-specific index sequence. To reduce the risk of bias and errors, the PCR reaction was limited to 15 cycles. The acquired PCR product was extracted from a 2% Agarose gel and purified with QIAquick gel extraction kit (Qiagen, Hilden, Germany). Finally, all samples were pooled in equal amounts to reach a total concentration of 10 nM. Sequencing was done in one flow cell of an Illumina MiSeq v2 instrument and conducted at the National Genomics Infrastructure (Stockholm, Sweden).

### Sequencing data analysis.

FASTQ files were analyzed using in-house software. First, correct sequences were sorted out; a sequence was considered to be correct if two recognition sites, nine bases upstream and nine bases downstream of the variable region, were found. Additionally, the distance between these two sites had to be of correct length and all bases were required to have a Phred quality score of ≥ 30. Subsequently, the number of each encountered gene variant among the correct sequences was calculated for each of the sequenced samples.

### Subcloning, production and purification of lead ADAPTs.

Based on the next generation sequencing results, twelve different enriched gene variants for each antigen were selected for further evaluation. The genes selected appeared in high frequency only in the tracks for that specific antigen and were spread over several different sequential clusters. Gene variants for the selections towards TNFα, CRP, PSA, REN and Insulin were synthesized by Thermo Fisher Scientific (Waltham, MA USA) and amplified using primers with flanking regions containing restriction sites for subcloning into a T7 inducible expression vector. Variants from the selection towards ANG and MYDGF were isolated as described in the following section. The final products were sequence verified using Sanger sequencing by Microsynth Seqlab (Göttingen, Germany). All variants were expressed in *E. coli* BL21*(DE3) cells and extracted by sonication. The resulting lysates were subjected to purification by affinity chromatography using a resin coupled with Human Serum Albumin (HSA) (Produced in-house).

### Development of a PCR based strategy to rescue individual clones.

Although genes from five of the seven selections were initially synthesized as described above, a more cost-efficient strategy based on standard PCR protocol was also developed and used to rescue clones from the selection towards ANG and MYDGF. One unique oligo covering the randomized positions in Helix 1 and another covering the randomized positions in Helix 2 were used as forward and reverse primers respectively to amplify the unique sequence and introduce restriction sites for cloning into an expression vector. The same T7 inducible expression vector as above was equipped with the third and non-randomized helix where the present inventors also introduced a HindIII restriction site for assembly of the full-length sequence of the unique ADAPT variant.

### Circular dichroism.

Secondary structure content, melting temperatures and ability to refold after denaturation was evaluated in a Chirascan circular dichroism spectrometer (Applied Photophysics, Surrey, UK). Samples were diluted in PBS to a concentration of 0.2 mg/ml and all analyses were done in a cell with an optical path length of 1 mm. Secondary structure content was evaluated by measuring the ellipticity from 260 to 195 nm at 20°C. The melting temperatures were determined by monitoring the signal at 221 nm while increasing the temperature from 20°C to 100°C at a rate of 5°C per minute. The refolding capability was assessed by repeating the secondary structure evaluation after the sample had been subjected to the heat-treatment.

### Evaluation of oligomeric state though Size-exclusion Chromatography.

The aggregation propensity of the ADAPT variants was evaluated through Size-Exclusion Chromatography (SEC). The experiments were performed on an ÄKTA Pure system equipped with a Superdex 755/150 column (GE Healthcare, Stockholm, Sweden). The column was equilibrated with PBS and elution profiles were acquired by injection of 25 ul purified protein (with concentrations ranging between 0.5-1 mg/ml) at a flow rate of 0.45 ml/min. A calibrant of four different proteins (Conalbumin 75 kDa, Carbonic Anhydrase 29 kDa, Ribonuclease A 13.7 kDa and Apopotinin 7.5 kDa) was used for comparison.

### Target binding analysis.

Sensor-based binding analysis was performed using a Biacore T200 system (GE Healthcare, Stockholm, Sweden). All analyses were conducted using CM-5 sensor chips coated with the target antigen, another irrelevant antigen and HSA. One surface was left as reference. Binding analysis was performed in PBS-T at 25°C. The ADAPT variants were diluted in PBS-T to 500 nM, 250 nM, 125 nM, 62.5 nM and 31.25 nM. The samples were injected at a flow rate of 30 ul/min for 120s followed by injection of PBS-T for 240s to monitor the dissociation. Surfaces were regenerated with 10 mM HCl. Kinetic parameters were determined using the Biacore Insight Evaluation software, assuming a 1:1 binding.

The simultaneous binding of ADAPT to albumin and the target antigen was evaluated either through a preincubation assay or a capture assay. The preincubation assay was performed by injecting ADAPT variants preincubated with 10x molar excess of HSA over the sensor chip with the same parameters as for the direct binding analysis. For the capture assay, 500 nM of the ADAPT variant was first captured on the HSA surface for 50 seconds with a flow rate of 10 µl/min followed by injection of 200 or 500 nM of the target antigen. The target antigen was injected at a flow rate of 30 ul/min for 120s followed by injection of PBS-T for 240s to monitor the dissociation. Surfaces were regenerated with 10 mM HCl.

### Evaluation of ternary complex formation though Size-exclusion Chromatography.

After demonstration of simultaneous binding of albumin and target antigen in SPR, a final characterization step was performed to confirm the formation of the ternary complex in solution. HSA, ADAPT and target antigen were mixed together in a 1:1:1 molar ratio at a total concentration of 50 µM and incubated for 1 hour in RT before injection onto a SEC column. The same amounts of each binding partner were also injected separately. The experiments were performed on an ÄKTA Pure system equipped with a Superdex 755/150 column (GE Healthcare, Stockholm, Sweden). The column was equilibrated with PBS and elution profiles were acquired by injection of 25 ul sample at a flow rate of 0.45 ml/min.

### EXAMPLES - RESULTS

For the purpose of selecting functional single-domain bispecific binders towards HSA as well as other target antigens, a standard workflow was established. Following phage display selection and next generation sequencing analysis, single clones were recovered either by synthesis or through a PCR-based method with unique oligos covering the randomized positions. To make sure that all final candidates would be functional in future applications, all protein variants were first evaluated in terms of structure and stability before finally being tested for binding towards the different target antigens as well as to HSA. Those that were produced, purified and passed all quality checks of the workflow are described below, and are summarized in Table 3.

**Table 3. Results from the characterization of the target-binding ADAPT variants, describing melting temperatures, ability to refold, ability to bind simultaneously with HSA, and determined kinetic constants. N.D means that the value is not determinable.**

| **ADAPT variant** | **Tm [°C]** | **Refold after heat treatment** | **Simultaneous binding to HSA** | **K_{D} [M] Target** | **K_{D} [M] HSA** |
|---|---|---|---|---|---|
| TNFα_05 | 50.00 | Yes | Yes | 2.5 × 10⁻⁹ | **1.8** × 10⁻⁸ |
| TNFα_09 | 59 | Yes | Yes | 3.6 × 10⁻⁹ | 9.5 × 10⁻⁹ |
| CRP_09 | 59 | Yes | No | **1.1** × 10⁻⁶ | **7.7** × 10⁻⁹ |
| CRP_23 | 68 | Yes | No | 1.9 × 10⁻⁶ | 2.3 × 10⁻⁸ |
| CRP_38 | 58 | Yes | No | 5.2 × 10⁻⁸ | 1.3 × 10⁻⁸ |
| CRP_48 | 67 | Yes | No | 6.2 × 10⁻⁸ | 1.5 × 10⁻⁸ |
| PSA_01 | 58 | Yes | Yes | **2.8** × 10⁻⁷ | **1.1** × 10⁻⁹ |
| PSA_05 | 52 | No | Yes | 3.0 × 10⁻⁷ | 3.3 × 10⁻⁹ |
| REN_01 | 63 | Yes | No | **7.6** × 10⁻⁸ | **2.3** × 10⁻⁸ |
| REN_40 | 52 | Yes | No | 4.1 × 10⁻⁷ | 5.5 × 10⁻⁹ |
| ANG_2 | 46 | Yes | Yes | **5.4** × 10⁻⁹ | 2.0 × 10⁻⁹ |
| ANG_6 | 43 | Yes | Yes | 1.0 × 10⁻¹¹ | 1.1 × 10⁻⁹ |
| ANG_13 | 38 | Yes | Yes | 2.8 × 10¹¹ | 2.8 × 10⁻⁹ |
| ANG_16 | 42 | Yes | Yes | 2.4 × 10⁻⁹ | 1.0 × 10⁻⁹ |
| MYDGF_07 | 61 | Yes | Yes | 2.3× 10⁻⁷ | **4.7** × 10⁻⁹ |
| MYDGF_08 | 56 | No | Yes | 1.8 × 10⁻⁷ | 1.2 × 10⁻⁸ |
| Insulin_01 | N.D | Yes | Yes | **9.5** × 10-7 | **3.9** × 10⁻⁹ |
| Insulin_02 | 44 | No | Yes | 6.7 × 10⁻⁷ | 1.5 × 10⁻⁹ |
| Insulin_03 | 34 | Yes | Yes | 1.3 × 10⁻⁶ | 3.1 × 10-9 |

### Construction of the phage display library.

With the aim of allowing for simultaneous binding of both the native ligand HSA as well as other clinically relevant targets, a novel combinatorial library was designed and created. ABD has previously been engineered for this purpose, resulting in the library shown in Figure 1B, with randomized positions spread over the first and the third helix.

That first library was successfully used to develop bispecific binders towards several targets. However, even though they all bound both their target and HSA separately, binding of HSA hindered simultaneous binding to the target antigen in all cases [13,15]. The present inventors hypothesized that the quite large and bulky HSA molecule might cause a steric hindrance around the binding surface of Helix 1 and 3 and believed that shifting the randomized binding surface to Helix 1 and 2 could entail new possibilities (Figure 1A). As the present inventors were concerned that mutating positions in Helix 2 close to the HSA binding site could affect the binding, the library was based on an affinity matured ABD known as ABD035 [20].

For the construction of the new library, a forward oligonucleotide covering Helix 1 included variations in positions according to Figure 1A and 1C with an equal distribution of all natural amino acids except Pro, Cys and Gly, while the reverse oligonucleotide covering Helix 2 was based on degenerate codons introducing NNK in positions 22, 23 and 26. The final reverse oligonucleotide covering the third helix contained only constant positions.

The randomized positions in Helix 1 and 2 were chosen based on the position of their side chains, which were suggested to be located at a sufficient distance from the HSA binding surface, as shown in the crystal structure described by Lejon et al [21]. In addition, the library design was also based on previous knowledge on residues that are not directly involved in the HSA binding as well as positions that had previously been successfully substituted [22]. Residues that were highly conserved in sequence alignments among homologous domains, and therefore thought to be of importance, were avoided. After assembly of the complete gene encoding ADAPT variants in an amount corresponding to 10¹⁴ individuals, the library was transformed into XL1 blue cells with a total diversity of 5x10⁹. The final naive library was sequenced with Illumina MiSeq, yielding more than 500 000 reads and showed a variation and an amino acid distribution consistent with the design parameters (Data not shown).

### Phage display selection and sequence evaluation.

Four rounds of panning were performed against four of the five biotinylated target antigens: bTNFα (19.5 kDa), bCRP (25.7 kDa), bPSA (29.5 kDa), and bREN (44.9 kDa). The selection towards Insulin (5.8 kDa) was sequenced already after two panning rounds. Next generation sequencing with barcoded adapter sequences enabled evaluation of sequence enrichment throughout the selections. The first four selections demonstrated enrichment of specific sequences as well as a strong consensus sequence after the fourth panning round, and enrichment was present also after the second panning round of the Insulin selection, although, as expected, not as distinct (data not shown). The selection towards TNFα (data not shown) serves as an example, where the amino acid distribution of the 100 most enriched sequences in the different panning rounds was studied. In this example, it can be observed that the even distribution of amino acids in the naive library changes towards certain preferred amino acids during the panning. In the same example, it can also be seen that the amino acid Proline is quite prominent in position 23 and 26 of Helix 2. Proline, known for its rigid structure and helix-breaking properties, together with the flexible Glycine and disulphide-forming Cysteine, were all excluded from the design of Helix 1. The present inventors hypothesized that these amino acids would not be as much of a problem in Helix 2 but comparing the different selection results they saw an unexpected enrichment of one or more of these three amino acids for all selections. Based on these results, a new library was designed, with an even distribution of the 17 remaining amino acids in both Helix 1 and 2. To evaluate the new version of the library, two additional selections were performed using the target antigens Angiogenin (ANG) (16.8 kDa) and myeloid-derived growth factor (MYDGF) (18.9 kDa). These selections resulted in enrichment of relevant sequences not containing Cys, Pro or Gly, as expected from the library design.

By comparing the amino acid enrichment in each position for the different selections (data not shown), it seems as the localization of the binding surface is somewhat different for the different target antigens. This is not very surprising since the target antigens are different in their molecular properties and one could assume that different amino acid positions might be variously accessible to the different target antigens.

Another interesting finding derived from the sequencing data was that in all selections regardless of target, position 6 showed a strong preference for hydrophobic amino acids (A, I, L, V and W) and a large portion of the sequences from the TNFα, PSA, CRP and ANG selections even contained the Valine present in the parental ABD035. These results suggest that it is possible to improve and re-design the library to be even more focused.

From the sequencing data, certain promising ADAPT variants were chosen for further characterization. Twelve variants from each selection towards TNFα, CRP, PSA, REN, ANG and MYDGF were synthesized and cloned into expression vectors for production and purification. The variants were chosen based on their relative frequency as well as them being spread over several sequential clusters. For the Insulin selection, an alternative strategy was used where the variants were selected already after two rounds of panning, encouraged by the fact that previous examples (data not shown) implies that enrichment is sufficient already at that early stage. Table 4 shows the top ranked clones from the MiSeq data derived from the second panning round in the selection towards Insulin. Four of these variants showed high sequence similarity and was therefore selected for further characterization.

**Table 4. Top ranked clones after two rounds of selection towards Insulin. Clones selected for further characterization are marked in bold.**

| Rank | Sequence (variable positions) | # sequences | % of total |
|---|---|---|---|
| **1** | **VI..AY..QY..AF..SD..K** | **148** | **0.114** |
| **2** | **AI..AY..QY..SF..RE..V** | **91** | **0.070** |
| 3 | NA..WV..LN..YD..SR..I | 88 | 0.068 |
| **4** | **AV..AY..KY..AF..NE..L** | **70** | **0.054** |
| 5 | IW..AS..VR..HR..YE..V | 55 | 0.042 |
| 6 | EQ..WA..MW..ST..IL..T | 32 | 0.025 |
| 7 | YR..LW..WV..KK..MA..F | 26 | 0.020 |
| 8 | FL..WA..NY..AR..LY..A | 24 | 0.018 |
| 9 | IW..VL..HS..WQ..YP..F | 22 | 0.017 |
| **10** | **QV..AY..KY..AF..MS..Q** | **22** | **0.017** |
| 11 | NA..WH..VN..YY..AR..L | 12 | 0.009 |
| 12 | LW..SV..RM..AD..LC..K | 10 | 0.008 |

A few of the other top ranked clones could be found also in other selections towards other targets and was therefore disregarded. This is likely a result of sequencing errors, target-unspecific enrichment or contamination during selection. Regardless of reason, comparing results from several selections towards different targets has proven to be a useful strategy to exclude non-target-specific sequences.

### Characterization of structure and stability of lead ADAPT variants.

All ADAPT variants that were produced in reasonable yields were evaluated with regard to their thermal stability and secondary structure content by circular dichroism. Candidates that demonstrated the expected alpha helical content with two characteristic dips around 222 nm and 208 nm (Fig. 3) were chosen for further characterization. The thermal stability of the variants varied with melting temperatures (Tm) ranging from 34°C to 67°C (Table 3), which means that some selected variants have an increased stability while others have a decreased stability compared to the parental molecule ABD035 that has a reported Tm of 58°C [20]. Several variants also had the ability to refold after heat denaturation (Table 3, Fig. 3). Those variants that demonstrated high alpha helical content were further assessed for their tendency to form unwanted oligomers. This was done by analysis on a size-exclusion chromatography column. Samples with single peaks (at the correct elution time compared to the standard) were regarded as being in a monomeric state (Fig. 4). Variants with peaks corresponding to larger complexes or degradation products were omitted from later characterizations.

### Evaluation of target binding, affinity and simultaneous binding to HSA.

Variants with sufficient alpha helical structure as well as monomeric elution profiles on the SEC column were further analyzed for binding towards their target protein as well as to HSA. The binding towards the target antigen used in the selection was measured by a multi-cycle analysis using SPR, showing that several binders had affinities in the µM-pM range. ADAPT variants were considered binders only if they demonstrated binding exclusively for the target molecule and not to other irrelevant target proteins. Analysis revealed 19 ADAPT variants that bound their target protein in a specific manner (the variants and their characteristics are summarized in Table 3). After target binding had been established, the variants were also evaluated for their ability to simultaneously bind to HSA. This was done either through an SPR capture assay or an experiment where the ADAPT was pre-incubated with saturating conditions of HSA before the binding towards the target was analyzed. Figure 2A shows a representative example of ADAPTInsulin_02 binding to both Insulin and HSA when injected on its own. Furthermore, the figure also shows that ADAPTInsulin_02 can still bind Insulin after being incubated with saturating concentrations of HSA, which is further validated by the fact that it can no longer bind HSA (Figure 2B). In total, thirteen of the characterized binders from the selections towards TNFα, PSA, ANG, MYDGF and Insulin could bind both HSA and its target antigen simultaneously. Figure 5 contains sensorgrams for the thirteen variants in the capture assay.

### Evaluation of ternary complex formation through Size-exclusion Chromatography.

As a final characterization, the ability to form the ternary complex consisting of the ADAPT variants together with albumin as well as the target antigen in solution was evaluated. ADAPTInsulin_02 together with its targets serves as an example of this in Figure 6. In this chromatogram, a small shift in elution profile can be seen when the ternary complex has been allowed to form, compared to both the injection of the binary complex as well as of the individual binding partners. The signal overlap is attributed to the limited resolution of the SEC column. Further strengthening the claim of complex formation, no free ADAPTInsulin_02 and only a small amount of free Insulin can be detected when injected as a complex. These data confirm what the present inventors could see in the SPR results, that the ADAPTs are not only bispecific, but they can also bind both albumin and their target at the same time.

### Discussion

Thus, the present inventors have designed a new combinatorial library with the aim of developing bispecific ADAPTs towards various new targets in addition to serum albumin. Through the use of phage display and next generation sequencing, they have identified and evaluated bispecific binders towards seven different targets and demonstrated simultaneous binding to albumin for five of these. These single-domain bispecific binders offer an opportunity of combining the advantages of small molecules, such as good tissue penetration and non-invasive administration routes, with those of much larger molecules that have a considerably longer *in vivo* half-life.

The results from this study confirm that a small 6 kDa domain can indeed accommodate two separate binding surfaces and hence bind to albumin without compromising either its size or the binding to its intended target. Furthermore, the present inventors have also developed an efficient workflow combining phage display with next generation sequencing to allow for the time- and cost-efficient selection of these binders towards many more targets in the future.

The binders, or bispecific proteins, were identified through the use of phage display in combination with Illumina high-throughput sequencing to gain a broader perspective on the enrichment process. The phage display technique has allowed for incredible discoveries and led to a great paradigm shift within the field of antibody development, but it is not without limitations. Performing repetitive panning rounds, each followed by an amplification step in bacterial cells, inevitably introduce bias. Furthermore, the high enrichment of one or a few sequences leads to a loss of sequence diversity and hence loss of some of the relevant variants that simply does not propagate as fast as others. Next generation sequencing is therefore particularly suitable for the application of phage displayed ADAPTs since one 150 bp read of the Illumina MiSeq covers the entire length of the randomized sequence. Also, by pooling together many individually barcoded samples in one single analysis, the present inventors have acquired hundreds of thousands of sequences from each of many different selections and panning rounds at a low cost. Another advantage of next generation sequencing is of course the depth of the data. Looking at the frequency of which the selected binders appear in their respective phage pools (Table 5), it can be seen that not all of these sequences have a fair chance of being discovered using traditional Sanger sequencing methods. In addition, by sequencing earlier panning rounds, the present inventors could see that the binding variants were sufficiently enriched to be discovered already after round 2 (data not shown). To test this in a real setting, the present inventors made a selection towards Insulin and picked four sequenced clones that appeared already after two panning rounds, which indeed resulted in three simultaneous binders. This confirms previously reported findings where positive clones have been identified without the need for tedious repetitive panning rounds [24,25]. This strategy, in particular, would not be possible without next generation sequencing since the variants binding to Insulin were only present in less than 0.1% of the total phage pool.

Despite the advantages of next generation sequencing, a major limitation of this workflow is the recovery of individual clones from the phage pool after identification. During this project the present inventors have mainly worked with synthesized gene fragments, which is not a very cost-efficient alternative if one wants to screen many variants. To overcome this, the present inventors later also developed a PCR based strategy where unique but shorter oligos are used to rescue individual clones. Taken together, the fact that positive clones can be discovered already after two panning rounds as well as recovered with a straight-forward PCR based method, leads to significant time and cost savings in the phage display process. It also removes some obstacles associated with performing many parallel selections in a multiplex and high-throughput manner.

Following characterization of the enriched variants it could be noted that several bispecific binders were identified in all seven different selections. The measured affinities were in the pM-µM range and with Angiogenin being the only exception, they are a result of relatively fast on-rates but also fast off-rates (Table 5). For the remaining molecules to be of major therapeutic value, affinity maturation will be needed in order to, most importantly, improve the off-rates. The present inventors believe that more stringent selection conditions could be one way to improve the off-rates and thus increase the affinities. The present inventors also think that the insight gained on the amino acid preferences in each position will help guiding in the construction of new and even more focused affinity maturation libraries. Furthermore, since the rationale behind choosing these ADAPT variants was that they appeared in different sequential clusters, another strategy could be to look deeper into the clusters where binding variants were identified.

Interestingly, even though bispecific binders were identified in all selections regardless of target antigen used, not all resulted in binders that could bind both their targets simultaneously. Simultaneous binders were identified for TNFα, PSA, ANG, MYDGF and Insulin but not for CRP and REN. Looking at the sizes of these targets, it is not evident that the molecular weight would influence the ability of simultaneous binding, even though it seems plausible that larger target antigens have a higher risk of causing sterical hindrance. However, it seems as if the preserved sequence regions of the simultaneous binders are shifted more towards Helix 1 while the non-simultaneous binders have more consensus also in Helix 2. Since the HSA binding surface is mainly located around Helix 2, this might explain why some bind simultaneously and others do not. With the increasing knowledge gained from these and future selections, the present inventors are confident that it will be possible to create a more focused library that will be even more successful in selecting not only numerous bispecific but also simultaneous binders.

As the present inventors expected, all variants bind HSA weaker than the parental ABD035. However, the affinities are still in the low nanomolar range and considering the very high HSA concentration in serum, these affinities are well above what is needed for efficient half-life extension. Indeed, previous studies have shown that the half-life extension is only weakly influenced by the affinity, even with affinities in the µM range [26].

**Table 5. Supplementary data for the target binding ADAPT variants. Frequency in sequencing data are derived from round 4 for TNFα, CRP, PSA, REN, ANG, MYDGF, and from round 2 for Insulin.**

| **ADAPT variant** | **Frequency in sequencing data [%]** | **K_{D} [M] Target** | **kₐ [M⁻¹s⁻¹] Target** | **k_{d} [s-1] Target** | **K_{D}[M] HSA** | **kₐ [M⁻¹s⁻¹] HSA** | **k_{d} [s-1] HSA** |
|---|---|---|---|---|---|---|---|
| TNFα_05 | 2.99 | 2.5e-09 | 1.5e+06 | 3.7e-03 | 1.8 × 10⁻⁸ | 2.1e+04 | 3.9e-04 |
| TNFα_09 | 43.4 | 3.6e-09 | 8.9e+05 | 3.2e-03 | 9.5e-08 | 3.6e+04 | 3.4e-04 |
| CRP_09 | 0.048 | 1.1e-06 | 1.2e+03 | 1.3e-03 | 7.76-09 | 4.9e+05 | 3.8e-03 |
| CRP_23 | 0.027 | 1.9e-06 | 8.9e+02 | 1.7e-03 | 2.3e-08 | 3.0e+05 | 7.0e-03 |
| CRP_38 | 1.884 | 5.2e-08 | 3.3e+04 | 1.7e-03 | 1.3e-08 | 4.1e+05 | 5.4e-03 |
| CRP_48 | 0.35 | 6.2e-08 | 1.9e+04 | 1.2e-03 | 1.5e-08 | 2.8e+05 | 4.2e-03 |
| PSA_01 | 44.9 | 2.8e-07 | 3.6e+05 | 1.0e-01 | 1.1e-09 | 1.1e+06 | 1.2e-03 |
| PSA_05 | 6.67 | 3.0e-07 | 2.0e+05 | 5-9e-02 | 3.3e-09 | 9.2e+04 | 3.0e-04 |
| REN_01 | 62.8 | 7.6e-08 | 3.2e+04 | 2.4e-03 | 2.3e-08 | 1.2e+04 | 2.7e-04 |
| REN_40 | 0.016 | 4.1e-07 | 8.7e+03 | 3.5e-03 | 5.5e-09 | 1.2e+05 | 6.4e-04 |
| ANG_02 | 0.495 | 1.4e-08 | 5.2e+04 | 7.0e-04 | 2.0e-09 | 2.3e+04 | 4.5e-05 |
| ANG_06 | 5.304 | 4.96-09 | 6.5e+04 | 3.2e-04 | 1.1e-09 | 2.4e+04 | 2.6e-05 |
| ANG_13 | 0.610 | 2.8e-11 | 1.1e+05 | 2.9e-06 | 2.8e-09 | 2.5e+04 | 6.8e-05 |
| ANG_16 | 0.329 | 5.1e-09 | 9.9e+04 | 5.0e-04 | 1.0e-09 | 2.9e+04 | 3.0e-05 |
| MYDGF_07 | 0.192 | 2.3e-07 | 3·5e+05 | 8.2e-02 | 4.7e-09 | 3.0e+04 | 1.4e-04 |
| MYDGF_08 | 2.172 | 1.8e-07 | 1.0e+06 | 1.8e-01 | 1.2e-08 | 2.3e+04 | 2.8e-04 |
| Insulin_01 | 0.070 | 9.5e-07 | 2.4e+05 | 2.2e-01 | 3.9e-09 | 3.6e+05 | 1.4e-03 |
| Insulin_02 | 0.017 | 6.7e-07 | 1.8e+05 | 1.2e-01 | 1.5e-09 | 6.4e+05 | 9.3e-04 |
| Insulin_03 | 0.054 | 1.3e-06 | 1.8e+05 | 2.2e-01 | 3.1e-09 | 4.6e+05 | 1.4e-03 |

### REFERENCES

[1] L. Urquhart, Top drugs and companies by sales in 2017, Nature. 17 (2018) 232.
[2] A.L. Grilo, A. Mantalaris, The Increasingly Human and Profitable Monoclonal Antibody Market, Trends Biotechnol. 37 (2019) 9-16. doi:10.1016/j.tibtech.2018.05.014.
[3] R. Bajracharya, J.G. Song, S.Y. Back, H.-K. Han, Recent Advancements in Non-Invasive Formulations for Protein Drug Delivery, Comput. Struct. Biotechnol. J. (2019). doi:10.1016/j.csbj.2019.09.004.
[4] M.D. Donovan, Y. Huang, Large molecule and particulate uptake in the nasal cavity: the effect of size on nasal absorption, 1998.
[5] A. Orlova, H. Wållberg, S. Stone-Elander, V. Tolmaehev, On the selection of a tracer for PET imaging of HER2-expressing tumors: Direct comparison of 124I-labeled affibody molecule and trastuzumab in a murine xenograft model, J. Nucl. Med. 50 (2009) 417-425. doi:10.2967/jnumed.108.057919·
[6] R. Goldstein, J. Sosabowski, M. Livanos, J. Leyton, K. Vigor, G. Bhavsar, G. Nagy-Davidescu, M. Rashid, E. Miranda, J. Yeung, B. Tolner, A. Plückthun, S. Mather, T. Meyer, K. Chester, Development of the designed ankyrin repeat protein (DARPin) G3 for HER2 molecular imaging, Eur. J. Nucl. Med. Mol. Imaging. 42 (2015) 288-301. doi:10.1007/s00259-014-2940-2.
[7] J. Garousi, S. Lindbo, J. Nilvebrant, M. Åstrand, J. Buijs, M. Sandström, H. Honarvar, A. Orlova, V. Tolmachev, S. Hober, ADAPT, a Novel Scaffold Protein-Based Probe for Radionuclide Imaging of Molecular Targets That Are Expressed in Disseminated Cancers, Cancer Res. 75 (2015) 4364-4371. doi:10.1158/0008-5472.CAN-14-3497.
[8] R.E. Kontermann, Strategies to Extend Plasma Half-Lives of Recombinant Antibodies, n.d.
[9] V. Tolmachev, A. Orlova, R. Pehrson, J. Galli, B. Baastrup, K. Andersson, M. Sandström, D. Rosik, J. Carlsson, H. Lundqvist, A. Wennborg, F.Y. Nilsson, Radionuclide Therapy of HER2-Positive Microxenografts Using a Lu-Labeled HER2-Specific Affibody Molecule, Cancer Res. 67 (2007) 2773-2782. doi:10.1158/0008-5472.CAN-06-1630.
[10] A. Orlova, A. Jonsson, D. Rosik, H. Lundqvist, M. Lindborg, L. Abrahmsen, C. Ekblad, F.Y. Frejd, V. Tolmachev, Site-specific radiometal labeling and improved biodistribution using ABY-027, a novel HER2-targeting affibody molecule-albumin-binding domain fusion protein, J. Nucl. Med. 54 (2013) 961-968. doi:10.2967/jnumed.112.110700.
[11] H. Liu, S. Lindbo, H. Ding, M. Altai, J. Garousi, A. Orlova, V. Tolmachev, S. Hober, T. Gräslund, Potent and specific fusion toxins consisting of a HER2-binding, ABD-derived affinity protein, fused to truncated versions of Pseudomonas exotoxin A, Int. J. Oncol. 55 (2019) 309-319. doi:10.3892/ijo.
[12] K.M.K. Sand, M. Bern, J. Nilsen, H.T. Noordzij, I. Sandlie, J.T. Andersen, Unraveling the Interaction between FcRn and Albumin: Opportunities for Design of Albumin-Based Therapeutics, Front. Immunol. 5 (2015) 682. doi:10.3389_{/}fimmu.2014.00682.
[13] T. Alm, L. Yderland, J. Nilvebrant, A. Halldin, S. Hober, A small bispecific protein selected for orthogonal affinity purification, Biotechnol. J. 5 (2010) 605-617. doi:10.1002/biot.201000041.
[14] J. Nilvebrant, T. Alm, S. Hober, J. Löfblom, Engineering Bispecificity into a Single Albumin-Binding Domain, PLoS One. 6 (2011). doi:10.1371/journal.pone.0025791.
[15] J. Nilvebrant, M. Åstrand, M. Georgieva-Kotseva, M. Björnmalm, J. Löfblom, S. Hober, Engineering of Bispecific Affinity Proteins with High Affinity for ERBB2 and Adaptable Binding to Albumin, PLoS One. 9 (2014). doi:10.1371/journal.pone.0103094.
[16] J. Nilvebrant, M. Åstrand, J. Löfblom, S. Hober, Development and characterization of small bispecific albumin-binding domains with high affinity for ErbB3, Cell. Mol. Life Sci. 70 (2013) 3973-3985. doi:10.1007/s00018-013-1370-9.
[17] A. Zorzi, S. Linciano, A. Angelini, Non-covalent albumin-binding ligands for extending the circulating half-life of small biotherapeutics, Med. Chem. Commun. 10 (2019) 1068. doi:10.1039/c9md00018f.
[18] C. Grönwall, A. Jonsson, S. Lindström, E. Gunneriusson, S. Stahl, N. Herne, Selection and characterization of Affibody ligands binding to Alzheimer amyloid β peptides, J. Biotechnol. 128 (2007) 162-183. doi:10.1016/j.jbiotec.2006.09.013.
[19] M. Uhlen, H. Tegel, Å. Sivertsson, C.-C. Kuo, J.M. Gutierrez, N.E. Lewis, B. Forsström, M. Dannemeyer, L. Fagerberg, M. Malm, H. Vunk, F. Edfors, A. Hober, E. Sjöstedt, D. Kotol, J. Mulder, A. Mardinoglu, J.M. Schwenk, P. Nilsson, M. Zwahlen, J. Ottosson Takanen, K. Von Feilitzen, C. Stadler, C. Lindskog, F. Ponten, J. Nielsen, B.O. Palsson, A.- Luisavolk, M. Lundqvist, A. Berling, A.-S. Svensson, S. Kanje, H. Enstedt, D. Afshari, S. Ekblad, J. Scheffel, B. Katona, J. Vuu, E. Lindström, L. Xu, R. Mihai, L. Bremer, M. Westin, M. Muse, L.M. Mayr, S. Knight, S. Göpel, R. Davies, P. Varley, D. Hatton, R. Fields, B.G. Voldborg, J. Rockberg, L.H. Schiavone, S. Hober, The human secretome - the proteins secreted from human cells, BioRxiv. (2018) 465815. doi:10.1101/465815.
[20] A. Jonsson, J. Dogan, N. Herne, L. Abrahmsén, P.Å. Nygren, Engineering of a femtomolar affinity binding protein to human serum albumin, Protein Eng. Des. Sel. 21 (2008) 515-527. doi:10.1093/protein/gzn028.
[21] S. Lejon, I.-M. Frick, L. Björck, S. Svensson, Crystal Structure and Biological Implications of a Bacterial Albumin Binding Module in Complex with Human Serum Albumin, J. Biol. Chem. 279 (2004) 42924-42928. doi:10.1074/jbc.M406957200.
[22] M.U. Johansson, I.-M. Frick, H. Nilsson, P.J. Kraulis, S. Hober, P. Jonasson, M. Linhult, P.-Å. Nygren, M. Uhlé, L. Bjö rck, T. rn Drakenberg, S. Forsé, M. Wikströ, Structure, Specificity, and Mode of Interaction for Bacterial Albumin-binding Modules*, (2001). doi:10.1074/jbc.M109943200.
[23] J.T. Andersen, R. Pehrson, V. Tolmachev, M.B. Daba, L. Abrahmsén, C. Ekblad, Extending half-life by indirect targeting of the neonatal Fc receptor (FcRn) using a minimal albumin binding domain, J. Biol. Chem. 286 (2011) 5234-5241. doi:10.1074/jbc.M110.164848.
[24] A. Christiansen, J. V Kringelum, C.S. Hansen, K.L. Bøgh, E. Sullivan, J. Patel, N.M. Rigby, T. Eiwegger, Z. Szépfalusi, F. De Masi, M. Nielsen, O. Lund, M. Dufva, High-throughput sequencing enhanced phage display enables the identification of patient-specific epitope motifs in serum, Nat. Publ. Gr. (2015). doi:10.1038/srep12913.
[25] P.A.C. 't Hoen, S.M.G. Jirka, B.R. ten Broeke, E.A. Schultes, B. Aguilera, K.H. Pang, H. Heemskerk, A. Aartsma-Rus, G.J. van Ommen, J.T. den Dunnen, Phage display screening without repetitious selection rounds, Anal. Biochem. 421 (2012) 622-631. doi:10.1016/J.AB.2011.11.005.
[26] J. Hopp, N. Hornig, K.A. Zettlitz, A. Schwarz, N. Fuβ, D. Mü, R.E. Kontermann, The effects of affinity and valency of an albumin-binding domain (ABD) on the half-life of a single-chain diabody-ABD fusion protein, Protein Eng. Des. Sel. 23 (2010) 827-834. doi:10.1093/protein/gzq058.
[27] J. Garousi, S. Lindbo, J. Borin, E. von Witting, A. Vorobyeva, M. Oroujeni, B. Mitran, A. Orlova, J. Buijs, V. Tolmachev, S. Hober, Comparative evaluation of dimeric and monomeric forms of ADAPT scaffold protein for targeting of HER2-expressing tumours, Eur. J. Pharm. Biopharm. 134 (2019) 37-48. doi:10.1016/j.ejpb.2018.11.004.
[28] V. Tolmachev, M. Friedman, M. Sandström, T.L.J. Eriksson, D. Rosik, M. Hodik, S. Stahl, F.Y. Frejd, A. Orlova, Affibody molecules for epidermal growth factor receptor targeting in vivo: Aspects of dimerization and labeling chemistry, J. Nucl. Med. 50 (2009) 274-283. doi:10.2967/jnumed.108.055525.
[29] Z. Li, B.F. Krippendorff, S. Sharma, A.C. Walz, T. Lavé, D.K. Shah, Influence of molecular size on tissue distribution of antibody fragments, MAbs. 8 (2016) 113-119. doi:10.1080/19420862.2015.1111497.
[30] H. Maeda, Tumor-Selective Delivery of Macromolecular Drugs via the EPR Effect: Background and Future Prospects, (2010). doi:10.1021/bc100070g.

## Claims

1. A bispecific protein capable of binding Human Serum Albumin (HSA) and another target, which bispecific protein comprises the amino acid sequence LAEAKVLANR X₁₁LDKX₁₅GX₁₇SX₁₉X₂₀ YKX₂₃LI X₂₆X₂₇AKT VEX₃₃VX₃₅X₃₆LX₃₈X₃₉X₄₀ ILX₄₃X₄₄X₄₅X₄₆ (SEQ ID No. 1), wherein:
X₁₁, X₁₅, X₁₇, X₁₉, X₂₇, X₃₃, X₃₅, X₃₆, X₃₈ and X₃₉ are, independently of each other, any amino acid;
X₄₃, X₄₄, X₄₅ and X₄₆ are, independently of each other, absent or any amino acid;
X₂₀ is Y or F;
X₂₃ is N, D or R;
X₂₆ is N or D; and
X₄₀ is E or H,
with the proviso that at least one of positions 22, 23 and 26 and at least one of positions 2, 3, 6, 7, 9, 10, 13 and 14 have been mutated to obtain the affinity for the other target.

2. The bispecific protein of claim 1, wherein at least two of positions 22, 23 and 26 have been mutated to obtain the affinity for the other target.

3. The bispecific protein of claim 1 or 2, wherein all of positions 22, 23 and 26 have been mutated to obtain the affinity for the other target.

4. The bispecific protein of any one of the preceding claims, wherein at least one of positions 2, 9 and 13 have been mutated to obtain the affinity for the other target.

5. The bispecific protein of any one of the preceding claims, wherein position 9 has been mutated to obtain the affinity for the other target.

6. The bispecific protein of any one of the preceding claims, wherein:
X₂₇ is N, K or D; and/or
X₃₃ is G, E, S or D.

7. The bispecific protein of any one of the preceding claims, wherein X₃₆ is D, A, S, E or K.

8. The bispecific protein of any one of the preceding claims, wherein neither of the amino acids in positions 1-15 are C.

9. The bispecific protein of any one of the preceding claims, wherein neither of the amino acids in positions 19-26 are C.

10. The bispecific protein of any one of the preceding claims, wherein neither of the amino acids in positions 31-44 are C.

11. The bispecific protein of any one of the preceding claims, wherein neither of the amino acids in positions 1-15 are G.

12. The bispecific protein of any one of the preceding claims, wherein neither of the amino acids in positions 19-26 are G.

13. The bispecific protein of any one of the preceding claims, wherein neither of the amino acids in positions 1-15 are P

14. The bispecific protein of any one of the preceding claims, wherein neither of the amino acids in positions 19-26 are P.

15. The bispecific protein of any one of the preceding claims, wherein neither of the amino acids in positions 31-44 are P.

16. The bispecific protein of any one of the preceding claims, wherein positions 1, 4, 5, 8, 12, 16, 25, 31, 34 and 42, each and independently, are conserved, or are conservatively substituted.

17. A polynucleotide encoding the bispecific protein according to any one of the claims 1-16.

18. An expression cassette comprising the polynucleotide according to claim 17.

19. A vector comprising the polynucleotide according to claim 16, or the expression cassette according to claim 17.

20. A pharmaceutical composition comprising the bispecific protein according to any one of the claims 1-16.

21. The pharmaceutical composition according to claim 20, wherein the target molecule is chosen from a group comprising of tumor necrosis factor alpha (TNFα), Prostate Specific Antigen (PSA), C-reactive protein (CRP), renin (REN), angiogenin (ANG), myeloid-derived growth factor (MYDGF) and insulin.

22. The pharmaceutical composition according any one of claims 20 or 21, further comprising a pharmaceutically acceptable carrier, excipient, stabilizer, additive, buffer solution, and/or solvent.

23. A therapeutic agent comprising the bispecific protein of any of the claims 1-16 and a cytotoxic agent, such as a cytotoxic molecule, peptide, protein or radionuclide.

24. The therapeutic agent according to claim 23, wherein the cytotoxic agent is an alkylating drug, anthracycline, an antimetabolite, a vinca alkaloide, an etoposide, an antineoplastic drug or agent, an exotoxin, a ribosome-inactivating protein, or a protein kinase inhibitor.

25. The therapeutic agent according to claim 23, wherein the cytotoxic radionuclide is selected from the group consisting of ¹⁷⁷Lu, ⁹⁰Y, ¹⁸⁸Re; ¹⁸⁶Re;¹⁶⁶Ho, ¹⁵³Sm, ⁶⁷Cu, ⁶⁴Cu,¹⁴⁹Tb, ¹⁶¹Tb, ⁴⁷Sc; ²²⁵Ac; ²¹²Pb; ²¹³Bi, ²¹²Bi, ²²⁷Th, ²²³Ra; ^{58m}Co, ¹³¹I, ⁷⁶As, ⁷⁷As and ²¹¹At.
